(19) Europäisches Patentamt
European Patent Office
Office européen des brevets

(11) EP 2 214 235 A1

(12) **EUROPEAN PATENT APPLICATION**
published in accordance with Art. 153(4) EPC

(43) Date of publication:
**04.08.2010 Bulletin 2010/31**

(21) Application number: **08845516.7**

(22) Date of filing: **24.10.2008**

(51) Int Cl.:
***H01M 4/58*** *(2010.01)* ***H01M 4/02*** *(2006.01)*
***H01M 4/50*** *(2010.01)* ***H01M 4/52*** *(2010.01)*
***H01M 10/40*** *(0000.00)*

(86) International application number:
**PCT/JP2008/069827**

(87) International publication number:
**WO 2009/057727 (07.05.2009 Gazette 2009/19)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.10.2007 JP 2007281358**
**18.04.2008 JP 2008108770**
**18.04.2008 JP 2008108771**

(71) Applicant: **Sumitomo Chemical Company, Limited**
**Tokyo 104-8260 (JP)**

(72) Inventors:
- **KIKUCHI, Taketoshi**
**Ibaraki-shi**
**Osaka 567-0841 (JP)**
- **KURAKANE, Kosuke**
**Ibaraki-shi**
**Osaka 567-0826 (JP)**
- **YAMAMOTO, Taketsugu**
**Tsukuba-shi**
**Ibaraki 305-0045 (JP)**
- **HATTORI, Takeshi**
**Abiko-shi**
**Chiba 270-1175 (JP)**
- **MAKIDERA, Masami**
**Tsukuba-shi**
**Ibaraki 305-0047 (JP)**

(74) Representative: **Hart-Davis, Jason et al**
**Cabinet Beau de Loménie**
**158, rue de l'Université**
**75340 Paris Cedex 07 (FR)**

# (54) NONAQUEOUS ELECTROLYTE SECONDARY BATTERY, ELECTRODE AND CARBON MATERIAL

(57) The present invention provides a nonaqueous electrolyte secondary battery, an electrode and a carbonaceous material. The nonaqueous electrolyte secondary battery has an electrode containing a carbonaceous material obtained by a production method comprising a step of heating a compound of formula (1) or a calcination product of the compound, under an inert gas atmosphere in the range of from 200°C to 3000°C:

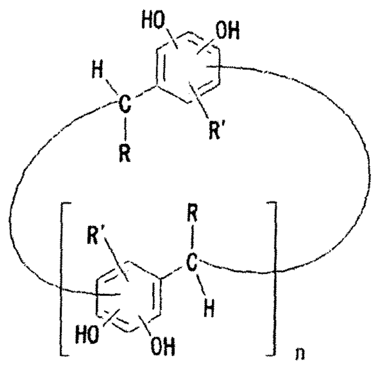

( 1 )

wherein R represents a hydrocarbon group having 1 to 12 carbon atoms, and a hydroxyl group, an alkyl group,

EP 2 214 235 A1

an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

FIG. 1

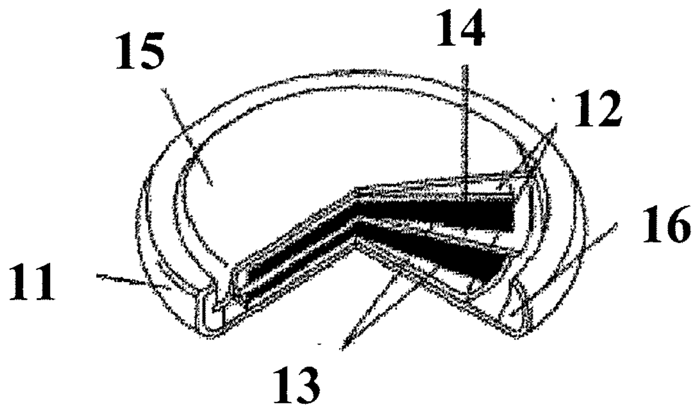

## Description

### Technical Field

The present invention relates to a nonaqueous electrolyte secondary battery, an electrode and a carbonaceous material. More particularly, the present invention relates to a nonaqueous electrolyte secondary battery such as a sodium ion secondary battery or the like, and to an electrode and a carbonaceous material used for the battery.

### Background Art

Recently, there is a desire for electric energy storage devices having a so-called excellent charge and discharge property showing no degradation of a charge and discharge capacity even after repetition of charge and discharge, in the field of charging type transport machines such as electric vehicles, and hybrid automobiles, the field of portable electronic terminals such as mobile personal computers, cellular telephones, and portable audio instruments, and the like. Specific examples of such electric energy storage devices include nonaqueous electrolyte secondary batteries such as a sodium ion secondary battery having electrodes, separator and electrolytic solution as main members, and the like (for example, JP-A No. 3-155062).

### Disclosure of the Invention

There was a situation for the electrode performance not to be necessarily sufficient by some kind of the electrolytic solution, some composition thereof and the like in the above-described sodium ion secondary battery. Under such a circumstance, it is an object of the present invention to provide a nonaqueous electrolyte secondary battery excellent in a charge and discharge property, particularly, a sodium ion secondary battery excellent in a charge and discharge property, and an electrode and a carbonaceous material used for the battery.

The present inventors have intensively investigated and finally completed the present invention.

That is, the present invention provides [1] to [13].

[1] A nonaqueous electrolyte secondary battery having an electrode containing a carbonaceous material obtained by a production method comprising a step of heating a compound of formula (1) or a calcination product of the compound, under an inert gas atmosphere in the range of from 200°C to 3000°C:

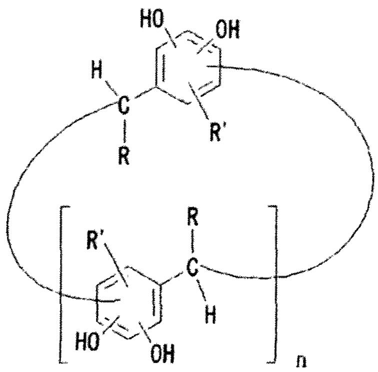

(1)

wherein R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

[2] The battery according to [1], wherein the nonaqueous electrolyte secondary battery is a sodium ion secondary battery.

[3] The battery according to [2], wherein R' in said formula (1) represents hydrogen.

[4] The battery according to [2] or [3], wherein the carbonaceous material has a total content of alkali metals and alkaline earth metals of 100 ppm or less (by weight).

[5] The battery according to any one of [2] to [4], wherein the compound of said formula (1) or the calcination product of the compound is obtained by heating the compound in the presence of an oxidizing gas at 400°C or lower.

[6] The battery according to any one of [2] to [5], wherein the carbonaceous material has a packing density of

from 0.5 to 0.8 g/cm$^3$.

[7] The battery according to any one of [2] to [6], wherein an electrolytic solution constituting the sodium ion secondary battery contains at least one ester solvent selected from the group consisting of propylene carbonate, ethylene carbonate, butylene carbonate, dimethyl carbonate, ethyl methyl carbonate, isopropyl methyl carbonate, vinylene carbonate and diethyl carbonate.

[8] The battery according to any one of [2] to [7], wherein the electrode containing the carbonaceous material is a negative electrode.

[9] The battery according to [8], wherein a positive electrode of the electrode contains a compound represented by $NaFeO_2$, $NaNiO_2$, $NaCoO_2$, $NaMnO_2$, $NaFe_{1-x}M^1{}_xO_2$, $NaNi_{1-x}M^1{}_xO_2$, $NaCo_{1-x}M^1{}_xO_2$, or $NaMn_{1-x}M^1{}_xO_2$ wherein $M^1$ represents one or more elements selected from the group consisting of trivalent metals, and $0 \leq x < 0.5$.

[10] The battery according to any one of [2] to [7], wherein the electrode containing the carbonaceous material is a positive electrode.

[11] The battery according to any one of [2] to [10], comprising a laminated porous film present between a positive electrode and a negative electrode, the laminated porous film having been obtained by laminating a heat resistant porous layer containing a heat resistant resin and a shutdown layer containing a thermoplastic resin.

[12] Use of a carbonaceous material obtained by a production method comprising a step of heating a compound of formula (1) or a calcination product of the compound, under an inert gas atmosphere in the range of from 200°C to 3000°C, for an electrode of a sodium ion secondary battery:

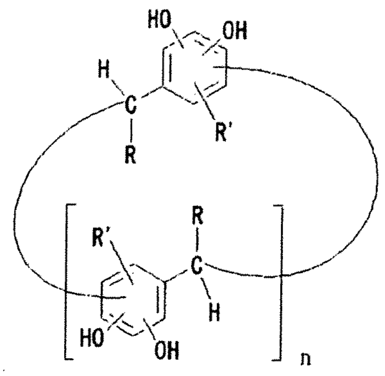

(1)

wherein R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

[13] Use of a compound of formula (1) or a calcination product of the compound, for producing an electrode composed of a carbonaceous material for a sodium ion secondary battery:

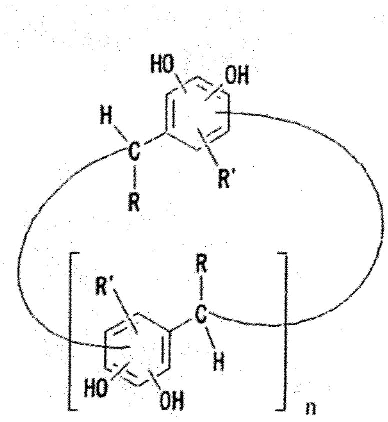

(1)

wherein R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

[14] A carbonaceous material obtained by a production method comprising a step of heating a compound of formula (1) or a calcination product of the compound, under an inert gas atmosphere in the range of from 1100°C to 2700°C:

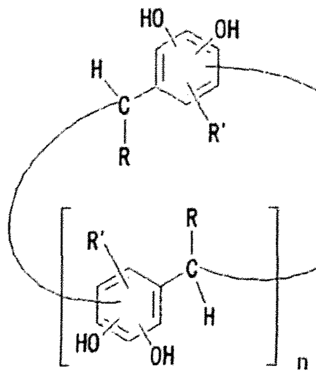

(1)

wherein R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

[15] The material according to [14], wherein R' in said formula (1) represents hydrogen.

[16] The material according to [14] or [15], wherein the carbonaceous material has a total content of alkali metals and alkaline earth metals of 100 ppm or less (by weight).

[17] The material according to any one of [14] to [16], wherein the compound of said formula (1) or the calcination product of the compound is a calcination product obtained by heating the compound in the presence of an oxidizing gas at 400°C or lower.

[18] An electrode containing the material according to any one of [14] to [17].

[19] Use of a carbonaceous material obtained by a production method comprising a step of heating a compound of formula (1) or a calcination product of the compound, under an inert gas atmosphere in the range of from 1100°C to 2700°C, for an electrode:

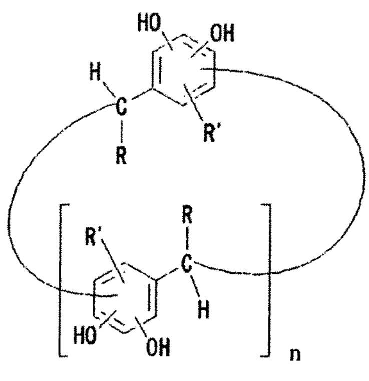

(1)

wherein R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

[20] A method of producing a carbonaceous material, comprising a step of heating a compound of formula (1) or a calcination product of the compound, under an inert gas atmosphere in the range of from 1100°C to 2700°C:

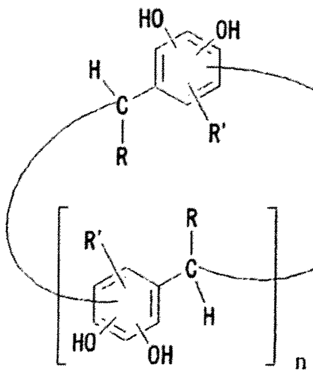

(1)

wherein R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

Brief Explanation of Drawings

Fig. 1 shows one example (schematic view) of a coin-shaped electric double layer.
Fig. 2 shows one example (schematic view) of a wound type electric double layer.
Fig. 3 shows one example (schematic view) of a laminated type electric double layer.
Fig. 4 shows one example (schematic view) of a laminated type electric double layer different from Fig. 3.
Fig. 5 shows a charge and discharge property in the battery of Example 1.
Fig. 6 shows a charge and discharge property in the battery of Example 3.

Explanation of marks

11 metal vessel, 12 current collector, 13 electrode, 14 separator, 15 metal lid, 16 gasket, 21 metal vessel, 22 current collector, 23 electrode, 24 separator, 25 electrode sealing plate, 26 lead, 31 metal vessel, 32 current collector, 33 electrode, 34 separator, 35 lead, 36 terminal, 37 safety valve, 41 pressing plate and terminal, 42 current collector, 43 electrode, 44 separator, 46 gasket, 51 pressing plate, 52 current collector, 53 electrode, 54 separator, 55 insulating material

Best Modes for Carrying Out the Invention

**Compound of formula (1) (referred to as compound (1))**

In formula (1), R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group.

Examples of R include alkyl groups such as a methyl group, an ethyl group, and a butyl group, cycloalkyl groups such as a cyclohexyl group, aromatic hydrocarbon groups such as a phenyl group, and a naphthyl group. Examples of the hydrocarbon group having the above-described substituent include aromatic hydrocarbon groups to which an alkyl group is attached such as a 2-tolyl group, a 3-tolyl group, and a 4-tolyl group, aromatic hydrocarbon groups to which a hydroxyl group is attached such as a 2-hydroxybenzyl group, a 3-hydroxybenzyl group, and a 4-hydroxybenzyl group. Particularly, from the standpoint of a yield in the carbonization, aromatic hydrocarbon groups to which the above-described substituent may be attached are preferable, and especially, aromatic hydrocarbon groups to which a hydroxyl group or an alkyl group may be attached are preferable.

R' in formula (1) represents a hydrogen atom or a methyl group, and a hydrogen atom is preferable from the standpoint of easiness of production.

In formula (1), n represents 3, 5 or 7, and from the standpoint of easiness of production, n represents preferably 3.

A hydroxyl group attached, together with R', to a benzene ring in formula (1) is usually attached to an ortho-position and para-position of -CH(R)-.

The compound (1) includes stereoisomers, and it may be only one stereoisomer of them or a mixture of stereoisomers. When the compound (1) is produced using an acid catalyst as described later, a mixture of stereoisomers is usually obtained.

Specific examples of the compound (1) include compounds of the following formula. Here, exemplified as R are groups shown on right side.

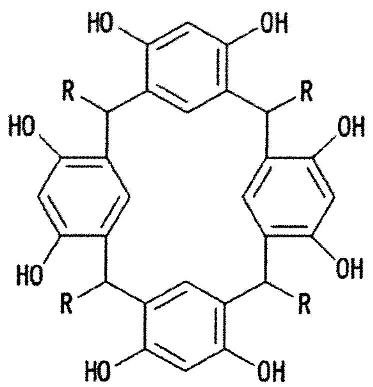

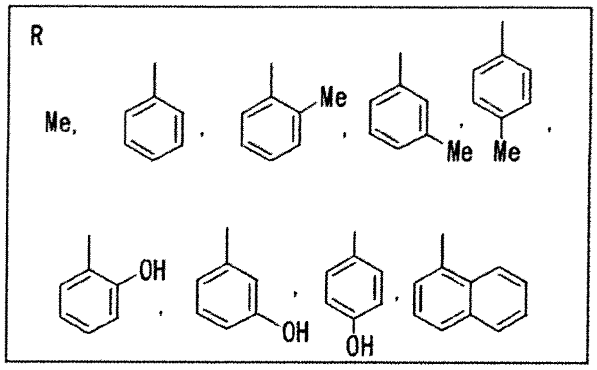

The compound (1) can be obtained, for example, from an aldehyde and resorcinol optionally having a methyl group and the like (hereinafter, referred to as resorcinols in some cases) as described in P. Timmerman et al., Tetrahedron, 52, (1996) p. 2663-2704.

Examples of the resorcinols to be used for production of the compound (1) include resorcinol, 2-methylresorcinol, and 5-methylresorcinol, and from the standpoint of easy availability, resorcinol is preferable.

Examples of the aldehyde to be used for production of the compound (1) include aliphatic aldehydes such as acetaldehyde, n-butyl aldehyde, isobutyl aldehyde, n-hexyl aldehyde, n-dodecyl aldehyde, 3-phenylpropionaldehyde, and 5-hydroxypentanal; and aromatic aldehydes such as benzaldehyde, 1-naphthoaldehyde, 2-methylbenzaldehyde, 3-methylbenzaldehyde, 4-methylbenzaldehyde, 2-hydroxybenzaldehyde, 3-hydroxybenzaldehyde, 4-hydroxybenzaldehyde, 4-t-butylbenzaldehyde, 4-phenylbenzaldehyde, 2-methoxybenzaldehyde, 3-methoxybenzaldehyde, 4-methoxybenzaldehyde, 2-chlorobenzaldehyde, 3-chlorobenzaldehyde, 4-chlorobenzaldehyde, 2-bromobenzaldehyde, 3-bromobenzaldehyde, 4-bromobenzaldehyde, 2-fluorobenzaldehyde, 3-fluorobenzaldehyde, 4-fluorobenzaldehyde, 2-methylthiobenzaldehyde, 3-methylthiobenzaldehyde, 4-methylthiobenzaldehyde, 2-carboxybenzaldehyde, 3-carboxybenzaldehyde, 4-carboxybenzaldehyde, 3-nitrobenzaldehyde, 4-aminobenzaldehyde, 4-acetylaminobenzaldehyde, and 4-cyanobenzaldehyde.

The use amount of the aldehyde is usually about 1 to 3 mol, and preferably about 1.2 to 2.5 mol with respect to 1 mol of resorcinols.

Examples of the acid catalyst to be used for production of the compound (1) include hydrochloric acid, sulfuric acid, phosphoric acid, nitric acid, and acetic acid, and particularly, hydrochloric acid or sulfuric acid is preferable. The use amount of the acid catalyst is usually about 0.001 to 3 mol with respect to 1 mol of resorcinols.

The aqueous solvent to be used for production of the compound (1) is water or a mixed solvent with water and an organic solvent which can be mixed with water at any proportion. Examples of the organic solvent include alcohol solvents such as methanol, ethanol, and i-propyl alcohol, ether solvents such as tetrahydrofuran, and the like. In the case of use of these solvents, these can be used singly or in admixture of two or more.

As the organic solvent used in the aqueous solvent, an alcohol solvent having 3 or less carbon atoms and a mixed solvent composed of water and an alcohol having 3 or less carbon atoms are preferable, and especially, an alcohol solvent having 3 or less carbon atoms is preferable.

Regarding the use amount ratio of the aqueous solvent and resorcinols, the amount of resorcinols is usually 0.5 to 5 parts by weight, preferably 1 to 2 parts by weight per 1 part by weight of the aqueous solvent.

Examples of a method of producing the compound (1) include a method in which resorcinols, an aldehyde, an acid catalyst and an aqueous solvent are mixed en bloc, and stirred usually at 0 to 100°C, preferably at 30 to 90°C, the compound (1) is allowed to deposit and the compound (1) is filtrated; a method in which an aldehyde is mixed, usually at 0 to 100°C, preferably at 30 to 90°C, into a mixture composed of resorcinols, an acid catalyst and an aqueous solvent, the compound (1) is allowed to deposit and the compound (1) is filtrated; a method in which resorcinols are mixed, usually at 0 to 100°C, preferably at 30 to 90°C, into a mixture composed of an aldehyde, an acid catalyst and an aqueous

solvent, the compound (1) is allowed to deposit and filtrated; a method in which an acid catalyst is mixed, usually at 0 to 100°C, preferably at 30 to 90°C, into a mixture composed of resorcinols, an aldehyde and an aqueous solvent, the compound (1) is allowed to deposit and filtrated; and the like.

In these production methods, a poor solvent such as water may be added before filtration of the compound (1).

The filtrated compound (1) is dried by a method such as through circulation drying, reduced pressure drying and the like usually at about 10°C to 100°C. The filtrated compound (1) may also be dried after substitution with a hydrophilic organic solvent. Here, examples of the hydrophilic organic solvent include alcohols such as methyl alcohol, ethyl alcohol, n-propyl alcohol, and t-butyl alcohol; aliphatic nitriles such as acetonitrile; aliphatic ketones such as acetone; aliphatic sulfoxides such as dimethyl sulfoxide; aliphatic carboxylic acids such as acetic acid.

## Carbonaceous material A

The carbonaceous material A can be obtained by a production method comprising a step A1 of heating a compound (1) or a calcination product of the compound, under an inert gas atmosphere in the range of from 200°C to 3000°C. Hereinafter, this step A1 is referred to as a carbonization step A1.

The method of the carbonization step A1 includes a method of heating under an atmosphere of a gas inactive to carbon (for example, nitrogen, and argon) in the range of from 200°C to 3000°C, preferably from 600°C to 2500°C, more preferably from 1100°C to 2700°C, further more preferably from 1500°C to 2500°C and particularly preferably from 1500°C to 2000°C for usually 1 minute to 24 hours.

Calcining at 200°C or higher is preferable, because there is a tendency of improvement of the packing density of the carbonaceous material A thereby. Calcining at 3000°C or lower is preferable, because there is a tendency of suppressing graphitization of the carbonaceous material A thereby.

As described above, the compound (1) may be immediately subjected to the carbonization step A1, however, it is also permissible that, for example, before the carbonization step A1, a step of heating the compound (1) in the presence of an oxidizing gas at 400°C or lower to generate a calcination product of the compound (1) (hereinafter, referred to as a pretreatment step A0) is carried out, subsequently, the oxidizing gas is replaced with an inert gas atmosphere and the carbonization step A1 is carried out.

Specifically, the compound (1) is heated in the presence of an oxidizing gas such as air, $H_2O$, $CO_2$, and $O_2$, preferably under an air atmosphere, at 400°C or lower, preferably in the range of from 200°C to 300°C for 1 minute to 24 hours. By the pretreatment step A0, an aggregated calcination product can be obtained, the product having enhanced molecular weight by crosslinkage of the compound (1) or the compound (1) partially carbonized.

Examples of the pretreatment step A0 and the carbonization step A1 include methods of heating using facilities such as a rotary kiln, roller hearth kiln, pusher kiln, multistage furnace, and fluidized furnace. In particular, a rotary kiln is capable of heating a large amount of compound (1) easily, and a powdery carbonaceous material A can be obtained without specific pulverization. In the method of producing the carbonaceous material A, the heating step does not include activation. The carbonaceous material A is different from an activated carbon.

The carbonaceous material A is in the form of a powder having a packing density of usually from 0.5 to 0.8 $g/cm^3$, preferably from 0.62 to 0.78 $g/cm^3$, particularly preferably from 0.65 to 0.75 $g/cm^3$. The packing density is measured according to a manual filling method described in JIS K1474 5.7.1 (packing density of carbonaceous material) using a 5 ml measuring cylinder.

The carbonaceous material ascribable to the compound (1) gives a powdery carbonaceous material A having the above-described packing density by a simple step without fractionation, as described later.

For the carbonaceous material A, it is preferable to adjust its average particle size to usually 50 $\mu$m or less, preferably 30 $\mu$m or less, particularly 10 $\mu$m or less. For obtaining smaller particle size, a further pulverization step may be performed in addition to the pretreatment step A0 and the carbonization step A1. By pulverizing the carbonaceous material finely, the packing density of an electrode is improved and the internal resistance can be decreased. The average particle size means a volume average particle size obtained by dispersing a carbonaceous material with a neutral detergent-containing aqueous solution and measuring using Laser Diffraction Particle Size Analyzer SALD2000J (registered trade mark, manufactured by Shimadzu Corporation).

For pulverization, pulverizers for fine pulverization such as, for example, an impulsive friction pulverizer, centrifugal force pulverizer, ball mill (tube mill, compound mill, conical ball mill, rod mill), vibration mill, colloid mill, friction disk mill, and jet mill are suitably used.

It is general to carry out fracture in a ball mill, and in the case of use of a ball mill, it is preferable that balls and pulverization vessel are made of nonmetal materials such as alumina, and agate, for avoiding mixing of a metal powder.

The carbonaceous material A can be controlled to have a total content of alkali metals and alkaline earth metals of 100 ppm or less, unless pulverized by a machine made of a metal. An electrode containing the carbonaceous material A is preferable since polarization by the metal is reduced, leading to a tendency of increase of the charge and discharge capacity.

## Carbonaceous material B

The carbonaceous material B can be obtained by a production method comprising a step B1 of heating a compound (1) or a calcination product of the compound, under an inert gas atmosphere in the range of from 1100°C to 2700°C, preferably in the range of from 1500°C to 2500°C. Hereinafter, the step B1 is referred to as a carbonization step B1.

The method of the carbonization step B1 includes a method of heating under an atmosphere of a gas inactive to carbon (for example, nitrogen, and argon) in the range of from 1100°C to 2700°C, preferably from 1500°C to 2500°C and particularly preferably from 1500°C to 2000°C for usually 1 minute to 24 hours.

As described above, the compound (1) may be immediately subjected to the carbonization step B1, however, it is also permissible that, for example, before the carbonization step, a step of heating the compound (1) in the presence of an oxidizing gas at 400°C or lower to generate a calcination product of the compound (1) (hereinafter, referred to as a pretreatment step B0) is carried out, subsequently, the oxidizing gas is replaced with an inert gas atmosphere and the carbonization step is carried out.

Specifically, the compound (1) is heated in the presence of an oxidizing gas such as air, $H_2O$, $CO_2$, and $O_2$, preferably under an air atmosphere, at 400°C or lower, preferably in the range of from 200°C to 300°C for 1 minute to 24 hours. By the pretreatment step B0, an aggregated calcination product can be obtained, the product having enhanced molecular weight by crosslinkage of the compound (1) or the compound (1) partially carbonized.

Examples of the pretreatment step B0 and the carbonization step B1 include methods of heating using facilities such as a rotary kiln, roller hearth kiln, pusher kiln, multistage furnace, and fluidized furnace. In particular, a rotary kiln is capable of heating a large amount of compound (1) easily, and a powdery carbonaceous material B can be obtained without specific pulverization. In the method of producing the carbonaceous material B, the heating step does not include activation. The carbonaceous material B is different from activated carbon.

The resultant carbonaceous material B is in the form of a powder having a packing density of usually from 0.5 to 0.8 $g/cm^3$, preferably from 0.62 to 0.78 $g/cm^3$, particularly preferably from 0.65 to 0.75 $g/cm^3$. The packing density is measured according to a manual filling method described in JIS K1474 5.7.1 (packing density of carbonaceous material) using a 5 ml measuring cylinder.

The carbonaceous material ascribable to the compound (1) gives a powdery carbonaceous material B having the above-described packing density by a simple step without fractionation, as described later.

For the carbonaceous material B, it is preferable to adjust its average particle size to usually 50 $\mu$m or less, preferably 30 $\mu$m or less, particularly 10 $\mu$m or less. For obtaining smaller particle size, a further pulverization step may be performed in addition to the pretreatment step B0 and the carbonization step B1. By pulverizing the carbonaceous material finely, the packing density of an electrode is improved and the internal resistance can be decreased.

The average particle size means a volume average particle size obtained by dispersing a carbonaceous material with a neutral detergent-containing aqueous solution and measuring using Laser Diffraction Particle Size Analyzer SALD2000J (registered trade mark, manufactured by Shimadzu Corporation).

For pulverization, pulverizers for fine pulverization such as, for example, an impulsive friction pulverizer, centrifugal force pulverizer, ball mill (tube mill, compound mill, conical ball mill, rod mill), vibration mill, colloid mill, friction disk mill, and jet mill are suitably used.

It is general to carry out fracture in a ball mill, and in the case of use of a ball mill, it is preferable that balls and pulverization vessel are made of nonmetal materials such as alumina, and agate, for avoiding mixing of a metal powder.

The carbonaceous material B can be controlled to have a total content of alkali metals and alkaline earth metals of 100 ppm or less, unless pulverized by a machine made of a metal. An electrode containing the carbonaceous material B is preferable since polarization by the metal is reduced, leading to a tendency of increase of the charge and discharge capacity.

## Electrode

The electrode is composed of the carbonaceous material A or the carbonaceous material B. The carbonaceous material A is suitable for an electrode of a nonaqueous electrolyte secondary battery, preferably for an electrode of a sodium ion secondary battery, more preferably for a negative electrode of a sodium ion secondary battery. The carbonaceous material B is suitable for an electrode of electric energy storage devices, for example, for an electrode of a dry battery, redox capacitor, hybrid capacitor, and electric double layer capacitor, and for an electrode of nonaqueous electrolyte secondary batteries such as a sodium ion secondary battery, and particularly, suitable for an electrode of a sodium ion secondary battery from the standpoint of the charge and discharge capacity and the charge and discharge efficiency.

The redox capacitor is a device containing an active material in an electrode and storing electricity by a redox reaction as described, for example, in "Forefront of large capacity electric double layer capacitor (daiyouryou denki

nijuusou capacitor no saizensen) (edited by Hideo Tamura, publisher: NTS)" 3-rd chapter (p.141 or later). It has a constitution in which the same separator as used in a sodium ion secondary battery to be described later is sandwiched between two electrodes, and an electrolytic solution is filled in the device. In the present specification, the electrolytic solution means a mixture of an electrolyte and a solvent.

The active material used in the redox capacitor includes oxides and hydroxides of transition metals such as ruthenium, conductive polymers and the like. The electrode is allowed to contain 1 to 60% by weight of a single body of a carbonaceous material ascribable to the compound (1) or a mixture of this carbonaceous material and a conductive agent, and 2 to 30% by weight of a binder.

For the electrolytic solution for the redox capacitor, when an oxide or a hydroxide of a transition metal such as ruthenium is used as an active material, there are mentioned a sulfuric acid aqueous solution and conditions described in, for example, JP-A No. 2002-359155. When an organic acid is used as an electrolyte and an electrolytic solution dissolved in an organic solvent is used, there are mentioned conditions described in, for example, JP-A No. 2002-267860. When a conductive polymer is used as an active material, those which are dissolved in an organic solvent to cause dissociation may be used as an electrolyte, and examples thereof include lithium salts such as $LiBF_4$, $LiPF_6$, and $LiClO_4$. Particularly it is desirable to use $LiPF_6$ since it has high degree of ionization and shows good solubility. These electrolytes may be used each singly or two or more of them may be used together. The concentration of an electrolyte in an electrolytic solution is preferably 0.5 to 1.5 mol/L from the standpoint of excellent ion conductivity. It is preferable that the concentration of an electrolyte be 0.5 mol/L or more, because there is a tendency of improvement of charge and discharge capacity. It is preferable that the concentration of an electrolyte be 1.5 mol/L or less, because there is a tendency of lowering of the viscosity of an electrolytic solution and improvement of ion conductivity.

As the solvent contained in an electrolytic solution for the redox capacitor, organic polar solvents exemplified for a sodium ion secondary battery to be described later are preferably used. It is desirable to use an aprotic organic solvent, and for example, one of cyclic carbonates, chain carbonates, cyclic esters and the like or a mixed solvent composed of two or more of them can be used. The cyclic carbonates include ethylene carbonate, propylene carbonate and the like, the chain carbonates include dimethyl carbonate, diethyl carbonate, methyl ethyl carbonate and the like, and the cyclic esters include γ-butyrolactone, γ-valerolactone and the like, respectively. Any one of them can be used singly or two or more of them can be used in admixture. The electrolytic solution is required to have high permittivity to promote dissociation of an electrolyte, and to have low viscosity not to present ion movement, further, to have high electrochemical resistance. Therefore, in particular, carbonate are suitable as a solvent, and for examples, it is desirable to use ethylene carbonate and the like as the high permittivity solvent and diethyl carbonate and the like as the low viscosity solvent, respectively in admixture.

The hybrid capacitor is a device in which, in charging, lithium ions are inserted into an interlaminar space of carbon of graphite and the like in a negative electrode and anions of an electrolyte are drawn to the electrode surface in a positive electrode, to constitute an electric double layer, thereby attaining storage of electricity. It has a constitution in which the same electrode as a negative electrode of a lithium ion secondary battery is used as the negative electrode and the above-described electrode is used as the positive electrode, and the same separator as an aggregated one described later is sandwiched between the positive electrode and the negative electrode, and an electrolytic solution is filled in the device. As the negative electrode, those specifically described in "Next generation lithium secondary battery (jisedai gata lithium niji denchi)(edited by Hideo Tamura, publisher: NTS)", 1-st chapter, 3-rd section (p 25 and later) can be used.

As the electrolyte for the hybrid capacitor, a combination of an inorganic anion and a lithium cation is usually used, and especially preferable is a combination of at least one inorganic anion selected from the group consisting of $BF_4^-$, $PF_6^-$ and $ClO_4^-$ and a lithium cation.

As the organic polar solvent contained in an electrolytic solution of the hybrid capacitor, solvents containing as the main component at least one selected from the group consisting of carbonates and lactones are usually used. Specifically exemplified solvents are cyclic carbonates such as propylene carbonate, ethylene carbonate, and butylene carbonate, chain carbonates such as dimethyl carbonate, ethyl methyl carbonate, and diethyl carbonate, or γ-butyrolactone and the like, and preferable solvents are a mixed solvent composed of ethylene carbonate and at least one of chain carbonates; a single body of γ-butyrolactone; a mixed solvent composed of γ-butyrolactone and at least one of chain carbonates; and the like.

**Secondary battery**

An electrode containing the carbonaceous material A is used for an electrode of a nonaqueous electrolyte secondary battery, preferably for an electrode of a sodium ion secondary battery, more preferably for a negative electrode of a sodium ion secondary battery. Further, an electrode containing the carbonaceous material B can be used for an electrode of an electric energy storage device, preferably for an electrode of a sodium ion secondary battery, more preferably for a negative electrode of a sodium ion secondary battery. The sodium ion secondary battery will be illustrated

in detail below. Usually, the sodium ion secondary battery can be produced as follows: a negative electrode, a separator and a positive electrode are laminated and wound to obtain an electrode group which is then accommodated in a vessel such as a battery can, then, it is impregnated with an electrolytic solution containing an electrolyte.

For producing the negative electrode of the sodium ion secondary battery, it is usual to mold a mixture containing a carbonaceous material ascribable to the compound (1) (carbonaceous material A or carbonaceous material B), a binder and, if necessary, a conductive agent and the like on a current collector. Specific examples thereof include a method in which a mixed slurry prepared by adding a solvent to the carbonaceous material ascribable to the compound (1) (carbonaceous material A or carbonaceous material B) and a binder and the like is applied on by a doctor blade method and the like or impregnated into a current collector and then dried; a method in which a solvent is added to the carbonaceous material ascribable to the compound (1) (carbonaceous material A or carbonaceous material B) and a binder and the like and the mixture is kneaded, molded and dried to obtain a sheet which is then joined to the surface of a current collector via a conductive adhesive and the like, then, subjected to pressing and thermal treatment and drying; a method in which a mixture composed of the carbonaceous material ascribable to the compound (1) (carbonaceous material A or carbonaceous material B), a binder, a liquid lubricant agent and the like is molded on a current collector, then, the liquid lubricant agent is removed, then, the resultant sheet-shaped molded article is subjected to a drawing treatment toward uniaxial or multiaxial directions; and the like. When the electrode is made into a sheet, its thickness is usually about from 5 to 500 $\mu$m.

Examples of the material of the current collector of a negative electrode include metals such as nickel, aluminum, titanium, copper, gold, silver, platinum, aluminum alloy, and stainless steel; those formed from a carbon material, activated carbon fiber, nickel, aluminum, zinc, copper, tin, lead or an alloy thereof by plasma thermal spray or arc thermal spray; conductive films obtained by dispersing a conductive agent in a rubber or a resin such as a styrene-ethylene-butylene-styrene copolymer (SEBS); etc. Particularly, copper is preferable since it scarcely forms an alloy with lithium and sodium, and it is easily processed into a thin film. Examples of the shape of the current collector include foil, flat plate, mesh, net, lath, punching or emboss, or a combination thereof (for example, meshed flat plate.) and the like. Irregularity may also be formed on the surface of a current collector by an etching treatment. As the binder, for example, polymers of fluorine compounds are mentioned. Examples of the fluorine compounds include fluorinated alkyl (having 1 to 18 carbon atoms) (meth)acrylate, perfluoroalkyl (meth)acrylate [for example, perfluorododecyl (meth)acrylate, perfluoro-n-octyl (meth)acrylate, perfluoro-n-butyl (meth)acrylatel, perfluoroalkyl substituted alkyl (meth)acrylate [for example, perfluorohexyl ethyl (meth)acrylate, perfluorooctyl ethyl (meth)acrylate], perfluorooxyalkyl (meth)acrylate [for example, perfluorododecyloxyethyl (meth)acrylate and perfluorodecyloxyethyl (meth)acrylate and the like], fluorinated alkyl (having 1 to 18 carbon atoms) crotonate, fluorinated alkyl(having 1 to 18 carbon atoms) malate and fumarate, fluorinated alkyl (having 1 to 18 carbon atoms) itaconate, fluorinated alkyl substituted olefin (having about 2 to 10 carbon atoms, having about 1 to 17 fluorine atoms), for example, perfluorohexylethylene; fluorinated olefin having about 2 to 10 carbon atoms and about 1 to 20 fluorine atoms in which a fluorine atom is attached to a double bond carbon; tetrafluoroethylene, trifluoroethylene, vinylidene fluoride or hexafluoropropylene and the like.

As other examples of the binder, addition polymers of monomers containing no fluorine atom and containing an ethylenic double bond are mentioned. Examples of such monomers include (meth)acrylate monomers such as (cyclo) alkyl (having 1 to 22 carbon atoms) (meth)acrylate [for example, methyl (meth)acrylate, ethyl (meth)acrylate, n-butyl (meth)acrylate, iso-butyl (meth)acrylate, cyclohexyl (meth)acrylate, 2-ethylhexyl (meth)acrylate, isodecyl (meth)acrylate, lauryl (meth)acrylate, and octadecyl (meth)acrylate]; aromatic ring-containing (meth)acrylate [for example, benzyl (meth) acrylate, and phenylethyl (meth)acrylate]; mono(meth)acrylate of alkylene glycol or dialkylene glycol (alkylene group having 2 to 4 carbon atoms) [for example, 2-hydroxyethyl (meth)acrylate, 2-hydroxypropyl (meth)acrylate, diethylene glycol mono(meth)acrylate]; (poly)glycerin (polymerization degree: 1 to 4) mono(meth)acrylate; poly-functional (meth) acrylate [for example, (poly)ethylene glycol (polymerization degree: 1 to 100) di(meth)acrylate, (poly)propylene glycol (polymerization degree: 1 to 100) di(meth)acrylate, 2,2-bis(4-hydroxyethylphenyl)propane di(meth)acrylate, trimethylolpropane and tri(meth)acrylate]: (meth)acrylamide monomers such as (meth)acrylamide, and (meth)acrylamide derivatives [for example, N-methylol(meth)acrylamide, and diacetone acrylamide]: cyano group-containing monomers such as (meth)acrylonitrile, 2-cyanoethyl (meth)acrylate, and 2-cyanoethylacrylamide: styrene monomers such as styrene and styrene derivatives having 7 to 18 carbon atoms [for example, $\alpha$-methylstyrene, vinyltoluene, p-hydroxystyrene, and divinylbenzene]: diene monomers such as alkane dienes having 4 to 12 carbon atoms [for example, butadiene, isoprene, and chloroprene]: alkenyl ester monomers such as carboxylic acid (having 2 to 12 carbon atoms) vinyl ester [for example, vinyl acetate, vinyl propionate, vinyl butyrate, and vinyl octanoate], and carboxylic acid (having 2 to 12 carbon atoms) (meth)allyl ester [for example, (meth)allyl acetate, (meth)allyl propionate, and (meth)allyl octanoate]: epoxy group-containing monomers such as glycidyl (meth)acrylate, and (meth)allyl glycidyl ether: monoolefins such as monoolefin having 2 to 12 carbon atoms [for example, ethylene, propylene, 1-butene, 1-octene, and 1-dodecene]: monomers containing a halogen atom other than fluorine such as chlorine, bromine or iodine atom-containing monomers, vinyl chloride, and vinylidene chloride: (meth)acrylic acid such as acrylic acid, and methacrylic acid: conjugated double bond-containing monomers such as butadiene, and isoprene: and the like. The addition polymer may also be a copolymer such as, for

example, an ethylene-vinyl acetate copolymer, styrenebutadiene copolymer, ethylene-propylene copolymer or the like. The vinyl carbonate polymer may be partially or completely saponified like polyvinyl alcohol. The binder may also be a copolymer composed of a fluorine compound and a monomer containing no fluorine atom and containing an ethylenic double bond.

Other examples of the binder include polysaccharides such as starch, methylcellulose, carboxymethylcellulose, hydroxymethylcellulose, hydroxyethylcellulose, hydroxypropylcellulose, carboxymethylhydroxyethylcellulose, and nitrocellulose, and derivatives thereof; phenol resin; melamine resin; polyurethane resin; urea resin; polyimide resin; polyamideimide resin; petroleum pitch; and coal pitch.

As the binder, polymers of fluorine compounds are preferable, and especially, polytetrafluoroethylene as a polymer of tetrafluoroethylene is preferable.

As the binder, several kinds of binders may be used.

The blending amount of the binder in an electrode is usually about from 0.5 to 30 parts by weight, preferably about from 2 to 30 parts by weight with respect to 100 parts by weight of a carbonaceous material ascribable to the compound (1).

Examples of the solvent used in the binder include aprotic polar solvents such as N-methylpyrrolidone; alcohols such as isopropyl alcohol, ethyl alcohol, and methyl alcohol; ethers such as propylene glycol dimethyl ether; and ketones such as acetone, methyl ethyl ketone, and methyl isobutyl ketone.

When the binder thickens, a plasticizer may be used for rendering application on a current collector easy.

The conductive adhesive is a mixture of a conductive agent and the above-described binder, and particularly, a mixture of carbon black and polyvinyl alcohol is suitable since there is no need to use an organic solvent, preparation thereof is easy, further, it is excellent also in storage ability.

To obtain the positive electrode of a sodium ion secondary battery, a mixture containing a positive electrode active material, a binder and, if necessary, a conductive agent and the like can be molded on a current collector by the same manner as for the negative electrode. As the binder and the conductive agent, the same materials as used for the negative electrode can be used. As the conductive agent, a carbonaceous material ascribable to the compound (1) (carbonaceous material A or carbonaceous material B) may be used.

As the positive electrode active material, materials which can be doped with sodium and dedopoed can be used. The materials which can be doped with sodium and dedopoed include compounds represented by $NaFeO_2$, $NaNiO_2$, $NaCoO_2$, $NaMnO_2$, $NaFe_{1-x}M^1{}_xO_2$, $NaNi_{1-x}M^1{}_xO_2$, $NaCo_{1-x}M^1{}_xO_2$, or $NaMn_{1-x}M^1{}_xO_2$, wherein $M^1$ represents one or more elements selected from the group consisting of trivalent metals, and $0 \leq x < 0.5$, and the like. In particular, by using a mixed oxide containing mainly iron and sodium and having a hexagonal crystal structure as the positive electrode active material, high discharge voltage can be obtained and a sodium ion secondary battery of high energy density can be obtained.

The positive electrode active material includes, further preferably, a mixed oxide containing mainly iron and sodium and having a hexagonal crystal structure which in X-ray diffraction analysis of the mixed oxide, the value obtained by dividing the peak intensity at a surface separation 2.20 Å by the peak intensity at a surface separation 5.36 Å is 2 or less. When a metal compound mixture containing a sodium compound and an iron compound is heated at a temperature in the range of 400°C or more and 900°C or less, it is preferable to carry out heating under an inert atmosphere in the temperature range of lower than 100°C during rising of the temperature.

As the current collector of the positive electrode, the same material as that of the negative electrode can be used, and particularly, aluminum, nickel, stainless steel and the like are preferable, especially, aluminum is preferable since it can be processed into a thin film easily and it is cheap.

Also a sodium ion secondary battery in which metal sodium is used as the negative electrode and an electrode containing a carbonaceous material ascribable to the compound (1) (carbonaceous material A or carbonaceous material B) is used as the positive electrode is regarded as a sodium ion secondary battery in the present specification. In the sodium ion secondary battery, charge and discharge are repeated by movement of sodium ions between the electrodes. In this case, the positive electrode is prepared in the same manner as for the above-described negative electrode.

The separator to be used in a nonaqueous electrolyte secondary battery such as a sodium ion secondary battery plays a role of separating the positive electrode and the negative electrode and holding an electrolytic solution, thus, an insulating membrane having a large ion permeability and having given mechanical strength is used as the separator.

As the separator, for example, materials made of a raw material such as a polyolefin resin such as polyethylene, polypropylene or the like, a fluorine resin, and a nitrogen-containing aromatic polymer, and having a form of porous membrane, nonwoven fabric, woven fabric and the like can be used. Two or more of the above-described raw materials may be used to give a separator, and the above-described materials may be laminated. Examples of the separator include those described in JP-A No. 2000-30686, JP-A No. 10-324758 and the like.

The pore size of the separator is usually about from 0.01 to 10 μm. The thickness of the separator is advantageously thinner from the standpoint of increase in the volume energy density of the battery and decrease in the internal

resistance thereof, providing that the mechanical strength is maintained, and it is usually about from 1 to 300 μm, preferably about from 5 to 40 μm.

The separator may be one obtained by laminating separators having different porosities.

For a nonaqueous electrolyte secondary battery such as a sodium ion secondary battery, it is usually preferable to have a function of interrupting electric current to block (shutdown) the flow of excessive current, when abnormal current flows in the battery due to short circuit between the positive electrode and the negative electrode, and the like. Shutdown means an operation by which, in the case of over usual use temperature, micropores of the separator are obstructed at as lower temperature as possible, and after obstruction, even if the temperature in the battery increases up to a certain high temperature, the separator is not broken at this temperature, and the condition of obstruction of micropores of the separator is maintained.

Examples of such a separator include a laminated porous film obtained by laminating a heat resistant porous layer containing a heat resistant resin and a shutdown layer containing a thermoplastic resin, and by this film, it becomes possible to further enhance the heat resistance of a nonaqueous electrolyte secondary battery such as a sodium ion secondary battery.

Next, the above-described laminated porous film obtained by laminating a heat resistant porous layer containing a heat resistant resin and a shutdown layer containing a thermoplastic resin will be illustrated, as an embodiment of the separator.

Example of the heat resistant resin to be used in the heat resistant porous layer include polyamides such as aromatic polyamides (para-oriented aromatic polyamide, meta-oriented aromatic polyamide), polyimides such as aromatic polyimides, polyamideimides such as aromatic polyamideimides, polyether sulfone, polyether imide, polycarbonate, polyacetal, polysulfone, polyphenylene sulfide, polyether ether ketone, aromatic polyester, polyether sulfone, polyether imide, poly-4-methylpentene-1 or cyclic olefin polymers.

For further enhancing heat resistance, nitrogen-containing aromatic polymers such as aromatic polyamides, aromatic polyimides, aromatic and polyamideimides are preferable, and particularly, para-oriented aromatic polyamides (hereinafter, referred to as "para-aramide" in some cases) are preferable from the standpoint of easy production.

The thermal membrane breakage temperature depends on the kind of the heat resistant resin in the heat resistant porous layer, and the thermal membrane breakage temperature is usually 160°C or more. Specifically, when the heat resistant resin is a nitrogen-containing aromatic polymer, the thermal membrane breakage temperature is at most about 400°C, in the case of use of poly-4-methylpentene-1, it is at most about 250°C, and in the case of use of a cyclic olefin polymer, it is at most about 300°C.

The para-aramide is obtained by condensation polymerization of a para-oriented aromatic diamine and a para-oriented aromatic dicarboxylic halide, and consists substantially of a repeating unit in which an amide bond is linked at a para-position or according orientation position of an aromatic ring (for example, orientation position extending coaxially or parallel toward the reverse direction, such as 4,4'-biphenylene, 1,5-naphthalene, 2,6-naphthalene and the like). Specifically exemplified are para-aramides having a para-orientation type structure or a structure according to the para-orientation type, such as poly(paraphenyleneterephthalamide), poly(para-benzamide), poly(4,4'-benzanilide terephthalamide), poly(para-phenylene-4,4'-biphenylene dicarboxylic amide), poly(para-phenylene-2,6-naphthalene dicarboxylic amide), poly(2-chloro-paraphenyleneterephthalamide), and paraphenyleneterephthalamide/2,6-dichloro paraphenyleneterephthalamide copolymer.

As the above-described aromatic polyimide, preferable are wholly aromatic polyimides produced by polycondensation of an aromatic dianhydride and a diamine. Specific examples of the dianhydride include pyromellitic dianhydride, 3,3',4,4'-diphenylsulfone tetracarboxylic dianhydride, 3,3',4,4'-benzophenone tetracarboxylic dianhydride, 2,2'-bis(3,4-dicarboxyphenyl)hexafluoropropane, and 3,3',4,4'-biphenyl tetracarboxylic dianhydride. Specific examples of the diamine include oxydianiline, para-phenylenediamine, benzophenonediamine, 3,3'-methylenedianiline, 3,3'-diaminobenzophenone, 3,3'-diaminodiphenylsulfone, and 1,5'-naphthalenediamine. Further, solvent-soluble polyimides can be suitably used. Examples of such a polyimide include a polyimide which is a polycondensate of 3,3',4,4'-diphenylsulfone tetracarboxylic dianhydride and an aromatic diamine.

The above-described aromatic polyamideimide includes those obtained by using an aromatic dicarboxylic acid and an aromatic diisocyanate and condensation-polymerizing them, and those obtained by using an aromatic dianhydride and an aromatic diisocyanate and condensation-polymerizing them. Specific examples of the aromatic dicarboxylic acid include isophthalic acid, and terephthalic acid. Specific examples of the aromatic dianhydride include trimellitic anhydride and the like. Specific examples of the aromatic diisocyanate include 4,4'-diphenylmethane diisocyanate, 2,4-tolylene diisocyanate, 2,6-tolylene diisocyanate, ortho-tolylane diisocyanate, and m-xylene diisocyanate.

For further enhancing ion permeability, it is preferable that the thickness of the heat resistant porous layer be 1 μm or more and 10 μm or less, further preferably 1 μm or more and 5 μm or less and particularly preferably 1 μm or more and 4 μm or less to be a thinner heat resistant porous layer. The heat resistant porous layer has micropores, and the pore size (diameter) is usually 3 μm or less, preferably 1 μm or less.

The heat resistant porous layer may also contain one or more kinds of fillers. A material of the filler may be

selected from any of an organic powder, an inorganic powder or a mixture thereof. It is preferable that particles constituting the filler have an average particle size of 0.01 μm or more and 1 μm or less.

Examples of the above-described organic powder include powders made of organic substances such as styrene, vinyl ketone, acrylonitrile, methyl methacrylate, ethyl methacrylate, glycidyl methacrylate, glycidyl acrylate, and methyl acrylate, singly or in the form of a copolymer composed of two or more of these compounds; fluorine resins such as polytetrafluoroethylene, ethylene tetrafluoride-propylene hexafluoride copolymer, ethylene tetrafluoride-ethylene copolymer, and polyvinylidene fluoride; melamine resins; urea resins; polyolefins; and polymethacrylate. These organic powders may be used singly, or in admixture of two or more. Among these organic powders, a polytetrafluoroethylene powder is preferable from the standpoint of chemical stability.

As the above-described inorganic powder, powders composed of inorganic substances such as, for example, metal oxides, metal nitrides, metal carbides, metal hydroxides, carbonates, and sulfates are mentioned. Specifically exemplified are powders composed of alumina, silica, titanium dioxide, calcium carbonate and the like. These inorganic powders may be used singly or in admixture of two or more. Among these inorganic powders, an alumina powder is preferable from the standpoint of chemical stability. Here, it is more preferable that all of particles constituting the filler be alumina particles, and further more preferable is an embodiment in which all of particles constituting the filler are alumina particles and a part of or all of them are approximately spherical alumina particles.

The content of a filler depends on the specific gravity of the material of the filler, and for example, when all of particles constituting the filler are alumina particles, the weight of the filler is usually 20 parts by weight or more and 95 parts by weight or less, preferably 30 parts by weight or more and 90 parts by weight or less, the total weight of the heat resistant porous layer being 100 parts by weight. These ranges can be appropriately set, depending on the specific gravity of the material of the filler.

The shape of the filler includes an approximately spherical shape, plate shape, column shape, needle shape, whisker shape, fiber shape and the like, and any particles can be used, and preferable are approximately spherical particles since uniform pores are formed easily with them.

In the above-described laminated porous film, the shutdown layer contains a thermoplastic resin. The shutdown layer has micropores like the above-described heat resistant porous layer, and the micropore size is usually 3 μm or less, preferably 1 μm or less. The shutdown layer has porosity of usually from 30 to 80% by volume, preferably from 40 to 70% by volume. In a sodium ion secondary battery, in the case of surpassing the usual use temperature, the shutdown layer plays a role of obstructing micropores due to softening of the thermoplastic resin constituting the layer.

As the above-described thermoplastic resin, those which are softened at from 80 to 180°C are mentioned, and those which are not dissolved in an electrolytic solution of a sodium ion secondary battery may be advantageously selected. Specifically mentioned are polyolefin resins such as polyethylene, and polypropylene, and thermoplastic polyurethane resins, and a mixture of two or more of these compounds may also be used. For softening at lower temperature to attain shutdown, polyethylene is preferable. As the polyethylene, specifically mentioned are ultrahigh molecular weight polyethylene, low density polyethylene, high density polyethylene, linear polyethylene and the like. For further enhancing the puncture strength of the shutdown layer, it is preferable that the thermoplastic resin contain at least an ultrahigh molecular weight polyethylene. From the standpoint of production of the shutdown layer, it is preferable in some cases that the thermoplastic resin contains a wax composed of a polyolefin of low molecular weight (weight average molecular weight of 10000 or less).

The thickness of the shutdown layer is usually from 3 to 30 μm, further preferably from 3 to 20 μm. The separator is obtained by lamination of the heat resistant porous layer and the shutdown layer, and the thickness of the separator is usually 40 μm or less, preferably 20 μm or less. It is preferable that the value of A/B is 0.1 or more and 1 or less, the thickness of the heat resistant porous layer being A (μm) and the thickness of the shutdown layer being B (μm).

In the sodium ion secondary battery, the separator has an air permeability according to the Gurley method of preferably from 50 to 300 sec/100 cc, further preferably from 50 to 200 sec/100 cc, from the standpoint of combination with ion permeability. The separator has a porosity of usually from 30 to 80% by volume, preferably from 40 to 70% by volume.

As the electrolyte used in the sodium ion secondary battery, sodium salts such as, for example, $NaClO_4$, $NaPF_6$, $NaBF_4$, $NacF_3SO_3$, $NaN (CF_3SO_2)_2$, $NaN(C_2F_5SO_2)_2$, and $NaC(CF_3SO_2)_3$ are used.

The concentration of the electrolyte may be appropriately set in view of the solubility of the electrolyte in an electrolytic solution, and it is usually about from 0.2 to 5 mol (electrolyte)/L (electrolytic solution), preferably about from 0.3 to 3 mol (electrolyte)/L (electrolytic solution), particularly preferably about from 0.8 to 1.5 mol/L mol (electrolyte)/L (electrolytic solution). It is preferable that the concentration be 0.2 mol/L or more, because there is a tendency of increase of the ion conductivity of the electrolytic solution and of lowering of the internal resistance of the sodium ion secondary battery. It is preferable that the concentration be 5 mol/L or less, because there is a tendency of lowering of the viscosity of the electrolytic solution and of lowering of the internal resistance of the sodium ion secondary battery.

As the solvent used in the electrolytic solution containing an electrolyte, organic polar solvents are used. The water content in the electrolytic solution containing an organic polar solvent is usually 200 ppm or less, preferably 50

ppm or less and further preferably 20 ppm or less. By restricting the water content in the electrolytic solution containing an organic polar solvent, an influence on an electrode by electrolysis of water, particularly, lowering of withstanding voltage, can be suppressed.

Specific examples of the organic polar solvent include the following compounds: (ether)

Monoethers (ethylene glycol monomethyl ether, ethylene glycol monoethyl ether, diethylene glycol monomethyl ether, diethylene glycol monoethyl ether, ethylene glycol monophenyl ether, tetrahydrofuran, 3-methyltetrahydrofuran and the like), diethers (ethylene glycol dimethyl ether, ethylene glycol diethyl ether, diethylene glycol dimethyl ether, diethylene glycol diethyl ether, diethyl ether, methyl isopropyl ether and the like), triethylene glycol dimethyl ether, ethylene glycol monomethyl ether acetate, cyclic ethers [(tetrahydrofuran, 2-methyltetrahydrofuran, 1,3-dioxolane, 1,4-dioxane, 2-methyl-1,3-dioxolane and the like) having 2 to 4 carbon atoms, 4-butyldioxolane, crown ether having 5 to 18 carbon atoms] and the like;

(fluorinated dioxolane)

2,2-di(trifluoromethyl)-1,3-dioxolane, 2,2-di(trifluoromethyl)-4,5-difluoro-1,3-dioxolane, 2,2-di(trifluoromethyl)-4,4,5,5-tetrafluoro-1,3-dioxolane, 2,2-dimethyl-4,4,5,5-tetrafluoro-2,3-dioxolane or 2,2-dimethyl-4,5-difluoro-1,3-dioxolane and the like;
(amide)
formamides (N-methylformamide, N,N-dimethylformamide, N-ethylformamide, N,N-diethylformamide and the like), acetamides (N-methylacetamide, N,N-dimethylacetamide, N-ethylacetamide, N,N-diethylacetamide and the like), propionamides (N,N-dimethylpropionamide and the like), hexamethylphosphorylamide and the like, oxazolidinones, N-methyl-2-oxazolidinone, 3,5-dimethyl-2-oxazolidinone and the like, 1,3-dimethyl-2-imidazolidinone, N-methylpyrrolidone and the like;
(nitrile)
acetonitrile, glutaronitrile, adiponitrile, methoxyacetonitrile, 3-methoxypropionitrile, acrylonitrile, fluorine-containing propionitrile obtained by substituting one or more hydrogen atoms of propionitrile by a fluorine atom, and the like;
(carboxylate)
methyl formate, ethyl formate, methyl acetate, ethyl acetate, propyl acetate, methyl propionate, methyl butyrate, methyl valerate, ethyl propionate, dimethyl malonate, diethyl malonate and the like, maleic anhydride and derivatives thereof, and the like;
(lactone)
γ-butyrolactone, 3-methyl-γ-butyrolactone, 2-methyl-γ-butyrolactone, α-acetyl-γ-butyrolactone, β-butyrolactone, γ-valerolactone, 3-methyl-γ-valerolactone, 5-valerolactone and the like;
(carbonate)
ethylene carbonate, propylene carbonate, butylene carbonate, vinylene carbonate, dimethyl carbonate, methyl ethyl carbonate, methyl propyl carbonate, methyl isopropyl carbonate, diethyl carbonate, 4-allyloxymethyl-1,3-dioxolan-2-one, 4-(1'-propenyloxymethyl)-1,3-dioxolan-2-one, 4-allyloxymethyl-5-vinyl-1,3-dioxolan-2-one, 4-(1'-propenyloxymethyl)-5-vinyl-1,3-dioxolan-2-one, 4-acryloyloxymethyl-1,3-dioxolan-2-one, 4-methacryloyloxymethyl-1,3-dioxolan-2-one, 4-methacryloyloxymethyl-5-vinyl-1,3-dioxolan-2-one, 4-methoxycarbonyloxymethyl-1,3-dioxolan-2-one, 4-allyloxycarbonyloxymethyl-1,3-dioxolan-2-one, 4-(1'-propenyloxycarbonyloxymethyl)-1,3-dioxolan-2-one, 4-vinyl-ethylene carbonate, 4,5-divinylethylene carbonate, 4,4,5,5-tetramethyl-1,3-dioxolan-2-one, 4,4,5,5-tetraethyl-1,3-dioxolan-2-one, vinylene carbonate, 4-methylvinylene carbonate, 4,5-dimethylvinylene carbonate, 5,5-dimethyl-1,3-dioxane-2-one and 5,5-diethyl-1,3-dioxan-2-one, dipropyl carbonate, methyl butyl carbonate, ethyl butyl carbonate, ethyl propyl carbonate, butyl propyl carbonate, compounds obtained by substituting one or more hydrogen atoms of the above-described compounds by a fluorine atom, and the like;
(sulfoxide)

dimethyl sulfoxide, sulfolane, 3-methylsulfolane, 2,4-dimethylsulfolane, fluorine-containing sulfolanes obtained by substituting one or more hydrogen atoms of sulfolane by a fluorine atom, and the like, 1,3-propane sultone, 1,4-butane sulfone, compounds obtained by substituting one or more hydrogen atoms by a fluorine atom, and the like;
(sulfone)
dimethylsulfone, diethylsulfone, di-n-propylsulfone, diisopropylsulfone, di-n-butylsulfone, di-sec-butylsulfone, di-tert-butylsulfone and the like;
(nitro compound)
nitromethane, nitroethane and the like;
(other heterocyclic compounds)
N-methyl-2-oxazolidinone, 3,5-dimethyl-2-oxazolidinone, 1,3-dimethyl-2-imidazolidinone, N-methylpyrrolidinone and the like;

(hydrocarbon)
aromatic solvents (toluene, xylene, ethylfluorobenzene, fluorobenzenes obtained by substituting 1 to 6 hydrogen atoms of benzene by fluorine atoms, and the like), paraffin solvents (n-paraffin, isoparaffin and the like), etc;
(silicon compound)
those having a silicon atom in the molecule, oxazolidinone compounds such as 3-trimethylsilyl-2-oxazolidinone, 3-trimethylsilyl-4-trifluoromethyl-2-oxazolidinone, and 3-triethylsilyl-2-oxazolidinone, imidazole compounds such as N-trimethylsilylimidazole, N-trimethylsilyl-4-methylimidazole, and N-triethylsilylimidazole, phosphate compounds such as tris (trimethylsilyl) phosphate, tris(triethylsilyl) phosphate, trimethylsilyldimethyl phosphate, and trimethylsilyldiallyl phosphate, cyclic carbonate compounds such as 4-trimethylsilyl-1,3-dioxolan-2-one, 4-trimethylsilyl-5-vinyl-1,3-dioxolan-2-one, and 4-trimethylsilylmethyl-1,3-dioxolan-2-one, phenyl compounds such as phenyltrimethylsilane, phenyltriethylsilane, phenyltrimethoxysilane, phenylthiotrimethylsilane, and phenylthiotriethylsilane, carbamate compounds such as methyl-N-trimethylsilyl carbamate, methyl-N,N-bistrimethylsilyl carbamate, ethyl-N-trimethylsilyl carbamate, methyl-N-triethylsilyl carbamate, and vinyl-N-trimethylsilyl carbamate, carbonate compounds such as methyltrimethylsilyl carbonate, allyltrimethylsilyl carbonate, and ethyltrimethylsilyl carbonate, methoxytrimethylsilane, hexamethyldisiloxane, pentamethyldisiloxane, methoxymethyltrimethylsilane, trimethylchlorosilane, butyldiphenylchlorosilane, trifluoromethyltrimethylsilane, acetyltrimethylsilane, 3-trimethylsilylcyclopentene, allyltrimethylsilane, vinyl-trimethylsilane, hexamethyldisilazane and the like.

The organic polar solvent for dissolving an electrolyte may be a mixture of different two or more solvents.

As the organic polar solvent contained in an electrolytic solution, particularly, solvents composed, as the main component, of ester solvents such as carbonates, and lactones are preferable, and in particular, ester solvents composed, as the main component, of at least one compound selected from the group consisting of propylene carbonate, ethylene carbonate, butylene carbonate, dimethyl carbonate, ethyl methyl carbonate, isopropyl methyl carbonate, vinylene carbonate and diethyl carbonate are preferable. Here, "as the main component" means that ester solvents occupy 50% by weight or more, preferably 70% by weight or more of solvents.

In conventional sodium ion secondary batteries containing an electrode, ether solvents are usually used, however, sodium ion secondary batteries containing the electrode are capable of performing charge and discharge even if ester solvents are used. Since ester solvents are excellent in oxidation resistance, sodium ion secondary batteries using an ester solvent as an electrolytic solution are capable of improving charge and discharge capacity (energy density) per unit volume as the battery by increasing the positive electrode voltage in operation. Further, there is a tendency of suppressing steep degradation of an electrolytic solution.

To the electrolytic solution, various additives can be added if necessary. Specifically mentioned are phosphates (trimethyl phosphate, triethyl phosphate, triallyl phosphate and the like), phosphonic acids and the like for suppressing gas generation and for improvement of withstanding voltage, and fluorine-containing organosilicon compounds of the following formula for obtaining higher capacity and higher power output.

$CF_3CH_2CH_2Si(CH_3)_3$

$(CH_3)_3Si\text{-}O\text{-}Si(CH_3)(CF_3CH_2CH_2)\text{-}Si(CH_3)$

The addition amount of the phosphate is usually about 10% by weight or less of an electrolyte and the addition amount of the fluorine-containing organosilicon compound is about from 0.1 to 5% by weight in an electrolytic solution, from the standpoint of the electric conductivity of the electrolyte and the solubility in the electrolytic solution.

Benzoic acids [for example, benzoic acid alkyl esters such as methyl benzoate, ethyl benzoate, propyl benzoate and the like, benzoic acid and the like] as the organic polar solvents may also be used as an additive for preventing elution of a metal from a current collector. When benzoic acids are as the additive, the addition amount thereof is usually about from 0.001 to 10.0% by weight, preferably from 0.005 to 5% by weight, particularly preferably from 0.1 to 1% by weight of an electrolyte.

Instead of the organic polar solvent, a solid electrolyte may also be used. The solid electrolyte is obtained by dispersing the above-described electrolyte in a resin, and the above-described organic polar solvent may further be dispersed therein. Specifically mentioned are gel electrolytes described in "Forefront of large capacity electric double layer capacitor (daiyouryou denki nijuusou capacitor no saizensen) (edited by Hideo Tamura, publisher: NTS)" p. 79, and solid electrolytes described in JP-A No. 2004-172346 and cited documents thereof, JP-A No. 2004-303567 and cited documents thereof, JP-A No. 2003-68580 and cited documents thereof, JP-A No. 2003-257240, and the like.

In the case of use of a solid electrolyte, the solid electrolyte plays a role of a separator, thus, a separator is not required in some cases.

Examples of the shape of the secondary battery include a coin shape, a wound shape, a laminated shape, and a bellow shape.

As the method of producing a coin-shaped battery, there is a method in which a current collector (12), electrode

(13), separator (14), electrode (13) and current collector (12) are laminated sequentially in a vessel (11) made of a metal such as stainless steel and the like, the vessel is filled with an electrolytic solution, then, sealed by a metal lid (15) and a gasket (16), as shown in Fig. 1.

As the method of producing a wound type battery, there is a method in which a mixed slurry containing the above-described carbonaceous material for electrodes is applied on a current collector (22) and dried, laminated sheets composed of the current collector (22) and an electrode (23) are prepared, the two sheets are wound via a separator (24), and housed together with an electrode sealing plate (25) into a cylindrical vessel (21) made of a metal such as aluminum, stainless steel and the like, as shown in Fig. 2.

The current collector is previously equipped with leads, and electricity is charged and discharged using a lead (26) of one laminated sheet as a positive electrode and a lead (26) of another laminated sheet as a negative electrode.

For the laminated shape, a method in which laminated sheets composed of a current collector (32) and an electrode (33), and separators (34) are laminated alternately, then, placed in a vessel (31) made of a metal such as aluminum, and stainless steel, an electrolytic solution is filled therein, the current collectors are connected to the leads (35) alternately, and the vessel is sealed, as shown in Fig. 3; a method in which laminated sheets composed of a current collector (42) and an electrode (43), and separators (44) are pressure-welded alternately, outer layers thereof are sealed with a rubber material and the like, an electrolytic solution is filled therein, then, the vessel is sealed, as shown in Fig. 4; and the like, are mentioned. A bipolar structure appropriately containing a gasket (46) by which use voltage can be set arbitrarily may also be permissible.

For the bellow shape, a method is mentioned in which two sheets composed of an electrode and a current collector are laminated while folding in the bellow form via a separator, then, preparation is made in the same manner as for the laminated shape.

A positive electrode and a negative electrode may be appropriately selected from electrodes in the drawing. Further, a bag like package composed of a laminated sheet containing aluminum and the like may also be used without using a metal hard case acting simultaneously as a negative electrode or a positive electrode terminal as an armoring.

A nonaqueous electrolyte secondary battery such as a sodium ion secondary battery is charged usually at a charging rate of from 0.1 to 20 hours, preferably at a charging rate of about from 0.5 to 5 hours. Applying at a charging rate of 20 hours or less is preferable, because there is a tendency of shortening of time necessary for charging. Charging at a charging rate of 0.1 hour or more is preferable, because there is a tendency of improvement of charge and discharge capacity.

A sodium ion secondary battery is preferable since even if quick charge and discharge are repeated at a charging rate of 5 hours or more, there is a tendency of suppression of lowering of charge and discharge capacity.

The time charging rate is a value (h) obtained by dividing charge and discharge capacity (mA·h) necessary for 100% charge by constant current value (mA) in charging, and larger this value, shorter the time for charging.

## EXAMPLES

Example 1: Heating at 1000°C (with a pretreatment step)

(Production Example of compound (1))

Production Example of tetraphenyl calix [4] resorcinarene (compound (1): PCRA)

Into a four-necked flask was charged 200 g of resorcinol, 1.5 L of methyl alcohol and 194 g of benzaldehyde under nitrogen flow and the flask was cooled with ice, and 36.8 g of 36% hydrochloric acid was dropped while stirring. After completion of dropping, the mixture was heated up to 65°C, then, thermally insulated for 5 hours at the temperature. To the resultant reaction mixture was added 1 L of water, a precipitate was filtrated, washed with water until the filtrate reached neutral, dried, then, 294 g of tetraphenyl calix [4] resorcinarene (PCRA) was obtained.

(Production Example 1 of carbonaceous material)

PCRA was heated at 300°C for 1 hour in a rotary kiln under an air atmosphere (procedure until this is a pretreatment step), then, the atmosphere in the rotary kiln was substituted by an argon atmosphere, and heating was performed under normal pressure at 1000°C for 4 hours. Then, the product was pulverized in a ball mill (agate balls, 28 rpm, 5 minutes) to obtain a powdery carbonaceous material. Since this powdery carbonaceous material is not in contact with metals, the content of metals including metal ions is 100 ppm or less. The resultant carbonaceous material has a packing density of 0.71 $g/cm^3$ and a BET specific surface area of 490 $m^2/g$.

Here, the BET specific surface area was measured from a value calculated from a nitrogen adsorption isothermal line at the liquid nitrogen temperature using AUTOSORB manufactured by Yuasa Ionics Inc.

Using the powdery carbonaceous material obtained in (Production Example 1 of carbonaceous material), a sodium ion secondary battery was fabricated as described below.

(1) Fabrication of electrode (positive electrode)

The carbonaceous material and a binder polyvinylidene fluoride (PVDF) were weighed so as to give a composition of carbonaceous material:binder = 95:5 (ratio by weight), the binder was dissolved in N-methylpyrrolidone (NMP), then, to this was added the carbonaceous material to obtain a slurry which was coated by a coating apparatus on a copper foil having a thickness of 10 μm as a current collector, and this was dried by a drier to obtain an electrode sheet. This electrode sheet was punched into a diameter of 1.5 cm with an electrode punching machine, to obtain a circular positive electrode (EA1).

(2) Fabrication of sodium ion secondary battery

The positive electrode was placed on a copper foil surface of a coin cell, 1M $NaClO_4$/(propylene carbonate) as an electrolytic solution was added, then, a polypropylene porous membrane was placed as a separator, further , metal sodium was placed as a negative electrode, to fabricate a sodium ion secondary battery TB1. Assembly of the battery was carried out in a glove box under an argon atmosphere.

(3) Charge and discharge test conditions

A charge and discharge test including repetition of charge and discharge at constant current under the following conditions was carried out.

Charge and discharge conditions:

Charging (doping of Na into carbon) was carried out at a constant current of 0.1 $mA/cm^2$ until 0.01 V.
Discharging (dedoping of Na from carbon) was carried out at a constant current of 0.1 $mA/cm^2$, and cut off at a voltage of 1.5 V.

(4) Results of charge and discharge test

The first discharge capacity was 260 mAh/g. In Fig. 5, the ordinate shows discharge capacity (mA·h/g) and the abscissa shows the number of repetition of the charge and discharge test (recycle number). As shown in Fig. 5, the discharge capacity at 10-th cycle was about 98% with respect to the discharge capacity at 5-the cycle, showing an excellent charge and discharge property. As a result of measurement of the charge and discharge curve at 10-th cycle, the charge capacity and the discharge capacity were approximately correspondent, and the charge and discharge efficiency was excellent. The average potential in discharging was 0.37 V.

Example 2

Using the carbonaceous material obtained in (Production Example 1 of carbonaceous material) in Example 1, a sodium ion secondary battery was fabricated as described below.

(1) Synthesis of positive electrode material

In a glove box under an argon atmosphere, $Na_2O_2$ (manufactured by Fluka Chemie AG) and $Fe_3O_4$ (manufactured by Aldrich Chemical Company, Inc.) were weighed so that Na and Fe showed a stoichiometric ratio of $NaFeO_2$, then, mixed thoroughly in an agate mortar. The resultant mixture was placed in an alumina crucible, and transferred into an electric furnace coupled to a glove box of which atmosphere had been previously exhausted by a vacuum pump before purging with argon introduced, and the atmosphere in the furnace was replaced with argon, and rising of temperature was initiated. Directly before reaching 100°C, the inside of the electric furnace was opened to air, thereafter, heating was carried out in an air atmosphere, maintained at 650°C for 12 hours, then, taken off to obtain a positive electrode material (MC1) for a sodium ion secondary battery. MC1 had a hexagonal crystal structure, and the value obtained by dividing the peak intensity at 2.20 Å by the peak intensity at a surface separation of 5.36 Å was 1.5.

(2) Fabrication of positive electrode

The positive electrode material (MC1) obtained in the above-described procedure (1) and a conductive agent

were weighed so as to give a composition of 70:25, mixed in an agate mortar, then, a binder polyvinylidene fluoride (PVDF) was weighed so as to give a composition of MC1:conductive agent:binder = 70:25:5 (ratio by weight), the binder was dissolved in N-methylpyrrolidone (NMP), then, to this was added a mixture of MC1 and the conductive agent to give a slurry which was then coated on an aluminum foil having a thickness of 10 μm as a current collector by a coating apparatus, and this was dried by a drier to obtain an electrode sheet. This electrode sheet was punched into a diameter of 1.4 cm by an electrode punching machine, to obtain a circular positive electrode (EC1).

(3) Fabrication of negative electrode

An electrode was fabricated in the same manner as for the positive electrode in Example 1, and this was used as a negative electrode (EA2).

(4) Fabrication of sodium ion secondary battery

On a lower lid of a coin cell, the positive electrode (EC1) was disposed with the aluminum foil surface facing downward, 1M $NaClO_4$/(propylene carbonate) was used as an electrolytic solution, a polypropylene porous membrane was used as a separator, further, the negative electrode (EA2) was placed with the copper foil surface facing upward, and these were combined to fabricate a sodium ion secondary battery TB2. Assembly of the sodium ion secondary battery was carried out in a glove box under an argon atmosphere. The resultant TB2 showed an excellent charge and discharge property.

Example 3 (Production Example 2 of carbonaceous material): heating at 2000°C (with a pretreatment step)

A powdery carbonaceous material was produced in the same manner as in Example 1 excepting that, for PCRA, the same pretreatment step was carried out as in Example 1, then, heating was performed at 2000°C for 1 hour in vacuo ($10^{-2}$ Pa) while flowing argon. The resultant carbonaceous material had a packing density of 0.50 g/cm$^3$ and a BET specific surface area of 1 m$^2$/g.

(1) Fabrication of electrode (positive electrode)

Using the resultant powdery carbonaceous material, a circular positive electrode (EA3) was obtained as described below in the same manner as in Example 1.

(2) Fabrication of sodium ion secondary battery

On a lower lid of a coin cell, the positive electrode was disposed with the copper foil surface facing downward, and 0.5M $NaClO_4$/(mixture of equal amount of ethylene carbonate and dimethyl carbonate) was used as an electrolytic solution, a polypropylene porous membrane was used as a separator, metal sodium was used as a negative electrode, and these were combined to fabricate a sodium ion secondary battery TB3. Assembly of the battery was carried out in a glove box under an argon atmosphere.

(3) Result of charge and discharge test

A charge and discharge test was carried out under the same conditions as in Example 1. In Fig. 6, the ordinate represents a ratio of the discharge capacity at each cycle after repetition of charge and discharge to the discharge capacity at 1-st cycle, the discharge capacity at 1-st discharge being 100%, and the abscissa represents the repetition number (recycle number) of the charge and discharge test. As shown in Fig. 6, the discharge capacity at 7-th cycle was about 99% with respect to the discharge capacity at 1-st cycle, showing an excellent charge and discharge property. As a result of measurement of a charge and discharge curve at 7-th cycle, the charge capacity and the discharge capacity were approximately correspondent, showing excellent charge and discharge efficiency. The potential in discharging was relatively flat and the average potential was low (the average potential in discharging was 0.19 V), and TB3 had high charge and discharge capacity and potential flatness, that is, this was a preferable sodium ion secondary battery.

Example 4

Using the carbonaceous material obtained in Production Example 2 of carbonaceous material, an electrode was fabricated in the same manner as for the positive electrode of Example 2, and this was used as a negative electrode (EA4), 0.5M $NaClO_4$/(mixture of equal amount of ethylene carbonate and dimethyl carbonate) was used as an electrolytic

solution, and the same procedures as in Example 2 were conducted excepting the above-described conditions, thereby fabricating a sodium ion secondary battery TB4. The resultant sodium ion secondary battery TB4 was subjected to a charge and discharge test, to find excellent charge and discharge efficiency.

Example 5 (Production Example 3 of carbonaceous material): heating at 1500°C (with a pretreatment step)

A powdery carbonaceous material was produced in the same manner as in Example 1 excepting that, for PCRA, the same pretreatment step was carried out as in Example 1, then, heating was performed at 1500°C for 1 hour. The resultant carbonaceous material had a BET specific surface area of 16 $m^2/g$.

(1) Fabrication of electrode (positive electrode)

Using the resultant powdery carbonaceous material, a circular positive electrode (EA5) was obtained as described below in the same manner as in Example 1.

(2) Fabrication of sodium ion secondary battery

On a lower lid of a coin cell, the positive electrode was disposed with the copper foil surface facing downward, and 1M $NaClO_4$/(propylene carbonate) was used as an electrolytic solution, a polypropylene porous membrane was used as a separator, metal sodium was used as a negative electrode, and these were combined to fabricate a sodium ion secondary battery TB5. Assembly of the battery was carried out in a glove box under an argon atmosphere.

(3) Result of charge and discharge test

A charge and discharge test was carried out under the same conditions as in Example 1. The discharge capacity at the first cycle was 300 mA/g. The discharge capacity at 10-th cycle was about 98% with respect to the discharge capacity at 5-th cycle, showing an excellent charge and discharge property. As a result of measurement of a charge and discharge curve at 10-th cycle, the charge capacity and the discharge capacity were approximately correspondent, showing excellent charge and discharge efficiency. The potential in discharging was relatively flat and the average potential was low (the average potential in discharging was 0.26 V), and TB5 had high charge and discharge capacity and potential flatness, that is, this was a preferable sodium ion secondary battery. The same effect of charge and discharge efficiency as for TB5 could be obtained, also by using 0.5M $NaClO_4$/(mixture of equal amount of ethylene carbonate and dimethyl carbonate) instead of 1M $NaClO_4$/(propylene carbonate) electrolytic solution, for TB5.

Example 6

Using the carbonaceous material obtained in Production Example 3 of carbonaceous material, an electrode was fabricated in the same manner as for the positive electrode of Example 2, and this was used as a negative electrode (EA6), 1M $NaClO_4$/(propylene carbonate) was used as an electrolytic solution, and the same procedures as in Example 2 were conducted excepting the above-described conditions, thereby fabricating a sodium ion secondary battery TB6. The resultant sodium ion secondary battery TB6 was subjected to a charge and discharge test, to find excellent charge and discharge efficiency.

Example 7 (Production Example 4 of carbonaceous material): heating at 1800°C (with a pretreatment step)

A powdery carbonaceous material was produced in the same manner as in Example 1 excepting that, for PCRA, the same pretreatment step was carried out as in Example 1, then, heating was performed at 1800°C for 1 hour. The resultant carbonaceous material had a BET specific surface area of 5 $m^2/g$.

(1) Fabrication of electrode (positive electrode)

Using the resultant powdery carbonaceous material, a circular positive electrode (EA7) was obtained as described below in the same manner as in Example 1.

(2) Fabrication of sodium ion secondary battery

On a lower lid of a coin cell, the positive electrode was disposed with the copper foil surface facing downward, and 1M $NaClO_4$/(propylene carbonate) was used as an electrolytic solution, a polypropylene porous membrane was

used as a separator, metal sodium was used as a negative electrode, and these were combined to fabricate a sodium ion secondary battery TB7. Assembly of the battery was carried out in a glove box under an argon atmosphere.

(3) Result of charge and discharge test

A charge and discharge test was carried out under the same conditions as in Example 1. The discharge capacity at the first cycle was 300 mA/g. The discharge capacity at 10-th cycle was about 99% with respect to the discharge capacity at 5-th cycle, showing an excellent charge and discharge property. As a result of measurement of a charge and discharge curve at 10-th cycle, the charge capacity and the discharge capacity were approximately correspondent, showing excellent charge and discharge efficiency. The potential in discharging was relatively flat and the average potential was low (the average potential in discharging was 0.24 V), and TB7 had high charge and discharge capacity and potential flatness, that is, this was a preferable sodium ion secondary battery. The same effect of charge and discharge efficiency as for TB7 could be obtained, also by using 0.5M $NaClO_4$/(mixture of equal amount of ethylene carbonate and dimethyl carbonate) instead of 1M $NaClO_4$/(propylene carbonate) electrolytic solution, for TB7.

Further, the same effect of charge and discharge efficiency as for TB7 could be obtained, also by using 0.1M $NaBF_4$/(propylene carbonate) and 0.1M $NaPF_6$/(propylene carbonate) as an electrolytic solution.

Example 8

Using the carbonaceous material obtained in Production Example 4 of carbonaceous material, an electrode was fabricated in the same manner as for the positive electrode of Example 2, and this was used as a negative electrode (EA8), 1M $NaClO_4$/(propylene carbonate) was used as an electrolytic solution, and the same procedures as in Example 2 were conducted excepting the above-described conditions, thereby fabricating a sodium ion secondary battery TB8. The resultant sodium ion secondary battery TB8 was subjected to a charge and discharge test, to find excellent charge and discharge efficiency.

Example 9 (Production Example 5 of carbonaceous material): heating at 2800°C (with a pretreatment step)

A powdery carbonaceous material was produced in the same manner as in Example 1 excepting that, for PCRA, the same pretreatment step was carried out as in Example 1, then, heating was performed at 2800°C for 1 hour. The resultant carbonaceous material had a BET specific surface area of 7 $m^2$/g.

(1) Fabrication of electrode (positive electrode)

Using the resultant powdery carbonaceous material, a circular positive electrode (EA9) was obtained as described below in the same manner as in Example 1.

(2) Fabrication of sodium ion secondary battery

On a lower lid of a coin cell, the positive electrode was disposed with the copper foil surface facing downward, and 0.5M $NaClO_4$/(mixture of equal amount of ethylene carbonate and dimethyl carbonate) was used as an electrolytic solution, a polypropylene porous membrane was used as a separator, metal sodium was used as a negative electrode, and these were combined to fabricate a sodium ion secondary battery TB9. Assembly of the battery was carried out in a glove box under an argon atmosphere.

(3) Result of charge and discharge test

A charge and discharge test was carried out under the same conditions as in Example 1. The discharge capacity at the first cycle was 84 mA/g. The discharge capacity at 10-th cycle was about 99% with respect to the discharge capacity at 5-th cycle, showing an excellent charge and discharge property. As a result of measurement of a charge and discharge curve at 10-th cycle, the charge capacity and the discharge capacity were approximately correspondent, showing excellent charge and discharge efficiency. The average potential in discharging was 0.32 V.

Example 10

Using the carbonaceous material obtained in Production Example 5 of carbonaceous material, an electrode was fabricated in the same manner as for the positive electrode of Example 2, and this was used as a negative electrode (EA10), 0.5M $NaClO_4$/(mixture of equal amount of ethylene carbonate and dimethyl carbonate) was used as an electrolytic

solution, and the same procedures as in Example 2 were conducted excepting the above-described conditions, thereby fabricating a sodium ion secondary battery TB10. The resultant sodium ion secondary battery TB10 was subjected to a charge and discharge test, to find excellent charge and discharge efficiency.

Example 11 (Production Example 6 of carbonaceous material): heating at 1000°C (without a pretreatment step)

A powdery carbonaceous material was produced in the same manner as in Example 1 excepting that, for PCRA, the pretreatment step was not used. The resultant carbonaceous material had a BET specific surface area of 673 $m^2/g$.

(1) Fabrication of electrode (positive electrode)

Using the resultant powdery carbonaceous material, a circular positive electrode (EA11) was obtained as described below in the same manner as in Example 1.

(2) Fabrication of sodium ion secondary battery

On a lower lid of a coin cell, the positive electrode was disposed with the copper foil surface facing downward, and 1M $NaClO_4$/(propylene carbonate) was used as an electrolytic solution, a polypropylene porous membrane was used as a separator, metal sodium was used as a negative electrode, and these were combined to fabricate a sodium ion secondary battery TB11. Assembly of the battery was carried out in a glove box under an argon atmosphere.

(3) Result of charge and discharge test

A charge and discharge test was carried out under the same conditions as in Example 1. The discharge capacity at the first cycle was 124 mA/g. The discharge capacity at 10-th cycle was about 99% with respect to the discharge capacity at 5-th cycle, showing an excellent charge and discharge property. As a result of measurement of a charge and discharge curve at 10-th cycle, the charge capacity and the discharge capacity were approximately correspondent, showing excellent charge and discharge efficiency. The average potential in discharging was 0.51 V. The same effect of charge and discharge efficiency as for TB11 could be obtained, also by using 0.5M $NaClO_4$/(mixture of equal amount of ethylene carbonate and dimethyl carbonate) instead of 1M $NaClO_4$/(propylene carbonate) electrolytic solution, for TB11.

Example 12

Using the carbonaceous material obtained in Production Example 6 of carbonaceous material, an electrode was fabricated in the same manner as for the positive electrode of Example 2, and this was used as a negative electrode (EA12), 1M $NaClO_4$/(propylene carbonate) was used as an electrolytic solution, and the same procedures as in Example 2 were conducted excepting the above-described conditions, thereby fabricating a sodium ion secondary battery TB12. The resultant sodium ion secondary battery TB12 was subjected to a charge and discharge test, to find excellent charge and discharge efficiency.

Example 13 (Production Example 7 of carbonaceous material): heating at 1500°C (without a pretreatment step)

A powdery carbonaceous material was produced in the same manner as in Example 1 excepting that, for PCRA, the pretreatment step was not used and heating at 1500°C was carried out for 1 hour. The resultant carbonaceous material had a BET specific surface area of 613 $m^2/g$.

(1) Fabrication of electrode (positive electrode)

Using the resultant powdery carbonaceous material, a circular positive electrode (EA13) was obtained as described below in the same manner as in Example 1.

(2) Fabrication of sodium ion secondary battery

On a lower lid of a coin cell, the positive electrode was disposed with the copper foil surface facing downward, and 1M $NaClO_4$/(propylene carbonate) was used as an electrolytic solution, a polypropylene porous membrane was used as a separator, metal sodium was used as a negative electrode, and these were combined to fabricate a sodium

ion secondary battery TB13. Assembly of the battery was carried out in a glove box under an argon atmosphere.

(3) Result of charge and discharge test

A charge and discharge test was carried out under the same conditions as in Example 1. The discharge capacity at the first cycle was 325 mA/g. The discharge capacity at 10-th cycle was about 99% with respect to the discharge capacity at 5-th cycle, showing an excellent charge and discharge property. As a result of measurement of a charge and discharge curve at 10-th cycle, the charge capacity and the discharge capacity were approximately correspondent, showing excellent charge and discharge efficiency. The potential in discharging was relatively flat and the average potential was low (the average potential in discharging was 0.26 V), and TB13 had high charge and discharge capacity and potential flatness, that is, this was a preferable sodium ion secondary battery. The same effect of charge and discharge efficiency as for TB13 could be obtained, also by using 0.5M $NaClO_4$/(mixture of equal amount of ethylene carbonate and dimethyl carbonate) instead of 1M $NaClO_4$/(propylene carbonate) electrolytic solution, for TB13.

Example 14

Using the carbonaceous material obtained in Production Example 7 of carbonaceous material, an electrode was fabricated in the same manner as for the positive electrode of Example 2, and this was used as a negative electrode (EA14), 1M $NaClO_4$/(propylene carbonate) was used as an electrolytic solution, and the same procedures as in Example 2 were conducted excepting the above-described conditions, thereby fabricating a sodium ion secondary battery TB14. The resultant sodium ion secondary battery TB14 was subjected to a charge and discharge test, to find excellent charge and discharge efficiency.

Example 15 (Production Example 8 of carbonaceous material): heating at 1800°C (without a pretreatment step)

A powdery carbonaceous material was produced in the same manner as in Example 1 excepting that, for PCRA, the pretreatment step was not used and heating at 1800°C was carried out for 1 hour. The resultant carbonaceous material had a BET specific surface area of 74 $m^2$/g.

(1) Fabrication of electrode (positive electrode)

Using the resultant powdery carbonaceous material, a circular positive electrode (EA15) was obtained as described below in the same manner as in Example 1.

(2) Fabrication of sodium ion secondary battery

On a lower lid of a coin cell, the positive electrode was disposed with the copper foil surface facing downward, and 1M $NaClO_4$/(propylene carbonate) was used as an electrolytic solution, a polypropylene porous membrane was used as a separator, metal sodium was used as a negative electrode, and these were combined to fabricate a sodium ion secondary battery TB15. Assembly of the battery was carried out in a glove box under an argon atmosphere.

(3) Result of charge and discharge test

A charge and discharge test was carried out under the same conditions as in Example 1. The discharge capacity at the first cycle was 291 mA/g. The discharge capacity at 10-th cycle was about 97% with respect to the discharge capacity at 5-th cycle, showing an excellent charge and discharge property. As a result of measurement of a charge and discharge curve at 10-th cycle, the charge capacity and the discharge capacity were approximately correspondent, showing excellent charge and discharge efficiency. The potential in discharging was relatively flat and the average potential was low (the average potential in discharging was 0.24 V), and TB15 had high charge and discharge capacity and potential flatness, that is, this was a preferable sodium ion secondary battery. The same effect of charge and discharge efficiency as for TB15 could be obtained, also by using 0.5M $NaClO_4$/(mixture of equal amount of ethylene carbonate and dimethyl carbonate) instead of 1M $NaClO_4$/(propylene carbonate) electrolytic solution, for TB15.

Example 16

Using the carbonaceous material obtained in Production Example 8 of carbonaceous material, an electrode was fabricated in the same manner as for the positive electrode of Example 2, and this was used as a negative electrode (EA16), 1M $NaClO_4$/(propylene carbonate) was used as an electrolytic solution, and the same procedures as in Example

2 were conducted excepting the above-described conditions, thereby fabricating a sodium ion secondary battery TB16. The resultant sodium ion secondary battery TB16 was subjected to a charge and discharge test, to find excellent charge and discharge efficiency.

Example 17 (Production Example 9 of carbonaceous material): heating at 2000°C (without a pretreatment step)

A powdery carbonaceous material was produced in the same manner as in Example 1 excepting that, for PCRA, the pretreatment step was not used and heating at 2000°C was carried out for 1 hour. The resultant carbonaceous material had a BET specific surface area of 1 $m^2/g$.

(1) Fabrication of electrode (positive electrode)

Using the resultant powdery carbonaceous material, a circular positive electrode (EA17) was obtained as described below in the same manner as in Example 1.

(2) Fabrication of sodium ion secondary battery

On a lower lid of a coin cell, the positive electrode was disposed with the copper foil surface facing downward, and 1M $NaClO_4$/(propylene carbonate) was used as an electrolytic solution, a polypropylene porous membrane was used as a separator, metal sodium was used as a negative electrode, and these were combined to fabricate a sodium ion secondary battery TB17. Assembly of the battery was carried out in a glove box under an argon atmosphere.

(3) Result of charge and discharge test

A charge and discharge test was carried out under the same conditions as in Example 1. The discharge capacity at the first cycle was 300 mA/g. The discharge capacity at 10-th cycle was about 98% with respect to the discharge capacity at 5-th cycle, showing an excellent charge and discharge property. As a result of measurement of a charge and discharge curve at 10-th cycle, the charge capacity and the discharge capacity were approximately correspondent, showing excellent charge and discharge efficiency. The potential in discharging was relatively flat and the average potential was low (the average potential in discharging was 0.17 V), and TB17 had high charge and discharge capacity and potential flatness, that is, this was a preferable sodium ion secondary battery. The same effect of charge and discharge efficiency as for TB17 could be obtained, also by using 0.5M $NaClO_4$/(mixture of equal amount of ethylene carbonate and dimethyl carbonate) instead of 1M $NaClO_4$/(propylene carbonate) electrolytic solution, for TB17.

Example 18

Using the carbonaceous material obtained in Production Example 9 of carbonaceous material, an electrode was fabricated in the same manner as for the positive electrode of Example 2, and this was used as a negative electrode (EA18), 1M $NaClO_4$/(propylene carbonate) was used as an electrolytic solution, and the same procedures as in Example 2 were conducted excepting the above-described conditions, thereby fabricating a sodium ion secondary battery TB18. The resultant sodium ion secondary battery TB18 was subjected to a charge and discharge test, to find excellent charge and discharge efficiency.

Example 19

REA1 was fabricated in the same manner as in Example 1 excepting that a conventional carbonaceous material (natural graphite) was used instead of the carbonaceous material EA1 used in the above-described Example 1, and using this REA1, a sodium ion secondary battery RTB1 was fabricated in the same manner as in Example 1. The resultant RTB1 was subjected to the same charge and discharge test as in Example 1. As a result, discharge capacity was scarcely obtained at 10-th cycle.

Production Example of laminated porous film

(1) Production of coating solution

Calcium chloride (272.7 g) was dissolved in NMP (4200 g), then, para-phenylenediamine (132.9 g) was added and dissolved completely. To the resultant solution was added gradually 243.3 g of terephthalic dichloride and polymerization thereof was carried out to obtain a para-aramide, and this was diluted further with NMP, to obtain a para-aramide solution (A) having a concentration of 2.0% by weight. To 100 g of the resultant para-aramide solution was added 2 g of an alumina powder (a) (manufactured by Nippon Aerosil Co., Ltd., Alumina C, average particle size 0.02 μm) and 2 g of an alumina powder (b) (Sumicorandom manufactured by Sumitomo Chemical Co., Ltd., AA03, average particle size 0.3 μm) as a filler in a total amount of 4 g, and these were mixed and treated three times by a nanomizer, and further, filtrated through a 1000 mesh wire netting, and de-foamed under reduced pressure to produce a slurry-formed coating solution (B). The weight of the alumina powders (filler) with respect to the total weight of the para-aramide and the alumina powders was 67% by weight.

(2) Production of laminated porous film and evaluation thereof

A polyethylene porous membrane (thickness 12 μm, air permeability 140 sec/100 cc, average pore size 0.1 μm, porosity 50%) was used as the shutdown layer. On a PET film having a thickness of 100 μm, the above-described polyethylene porous membrane was fixed, and the slurry-form coating solution (B) was coated on the porous membrane by a bar coater manufactured by Tester Sangyo Co., Ltd. The product was immersed in water as a poor solvent while maintaining integration of the porous membrane coated on the PET film, to cause deposition of a para-aramide porous membrane (heat resistant porous layer), then, the solvent was dried to obtain a laminated porous film 1 composed of a heat resistant porous layer and a shutdown layer laminated. The thickness of the laminated porous film 1 was 16 μm, and the thickness of the para-aramide porous membrane (heat resistant porous layer) was 4 μm. The laminated porous film 1 had an air permeability of 180 sec/100 cc, and a porosity of 50%. The cross section of the heat resistant porous layer in the laminated porous film 1 was observed by a scanning electron microscope (SEM) to find that relatively small micropores of about 0.03 μm to 0.06 μm and relatively large micropores of about 0.1 μm to 1 μm were present.
The sodium ion secondary battery using the above-described laminated porous film as a separator is capable of having further increased thermal breakage temperature.
Evaluation of the laminated porous film was carried out by the following method.

Evaluation of laminated porous film

(A) Measurement of thickness

The thickness of the laminated porous film and the thickness of the shutdown layer were measured according to JIS standard (K7130-1992). As the thickness of the heat resistant porous layer, a value obtained by subtracting the thickness of the shutdown layer from the thickness of the laminated porous film was used.

(B) Measurement of air permeability by Gurley method

The air permeability of the laminated porous film was measured by digital timer mode Gurley type Densometer manufactured by Yasuda Seiki Seisakusho Ltd., according to JIS P8117.

(C) Porosity

A sample of the resultant laminated porous film was cut into a square having a side length of 10 cm, and the weight W (g) and the thickness D (cm) thereof were measured. The weights (Wi) of the layers in the sample were measured, and the volumes of the respective layers were calculated from Wi and the true specific gravities (g/cm$^3$) of the raw materials of the respective layers, and the porosity (% by volume) was calculated according to the following formula.

$$\text{Porosity (\% by volume)} = 100\times\{1-(W1/\text{true specific gravity } 1+W2/\text{true specific gravity } 2+\cdot\cdot+Wn/\text{true specific gravity } n)/(10\times10\times D)\}$$

<u>Industrial Applicability</u>

According to the present invention, there are provided a nonaqueous electrolyte secondary battery excellent

in a charge and discharge property, particularly a sodium ion secondary battery excellent in a charge and discharge property, and a carbonaceous material used in them.

## Claims

1. A nonaqueous electrolyte secondary battery having an electrode containing a carbonaceous material obtained by a production method comprising a step of heating a compound of formula (1) or a calcination product of the compound, under an inert gas atmosphere in the range of from 200°C to 3000°C:

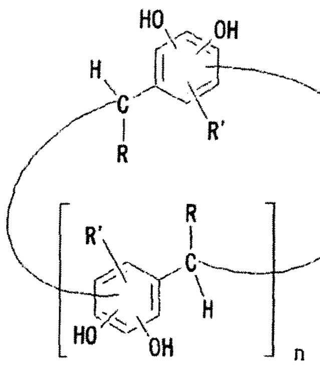

(1)

wherein R represents a hydrocarbon group having 1 to 12 carbon atoms, and a hydroxyl group, an alkyl group, an alkoxyl group, an aryl group, an aryloxy group, a sulfonyl group, a halogen atom, a nitro group, a thioalkyl group, a cyano group, a carboxyl group, an amino group or an amide group may be attached to the hydrocarbon group; R' represents a hydrogen atom or a methyl group; n represents 3, 5 or 7.

2. The battery according to Claim 1, wherein the nonaqueous electrolyte secondary battery is a sodium ion secondary battery.

3. The battery according to Claim 2, wherein R' in said formula (1) represents hydrogen.

4. The battery according to Claim 2 or 3, wherein the carbonaceous material has a total content of alkali metals and alkaline earth metals of 100 ppm or less (by weight).

5. The battery according to any one of Claims 2 to 4, wherein the compound of said formula (1) or the calcination product of the compound is obtained by heating the compound in the presence of an oxidizing gas at 400°C or lower.

6. The battery according to any one of Claims 2 to 5, wherein the carbonaceous material has a packing density of from 0.5 to 0.8 g/cm$^3$.

7. The battery according to any one of Claims 2 to 6, wherein an electrolytic solution constituting the sodium ion secondary battery contains at least one ester solvent selected from the group consisting of propylene carbonate, ethylene carbonate, butylene carbonate, dimethyl carbonate, ethyl methyl carbonate, isopropyl methyl carbonate, vinylene carbonate and diethyl carbonate.

8. The battery according to any one of Claims 2 to 7, wherein the electrode containing the carbonaceous material is a negative electrode.

9. The battery according to Claim 8, wherein a positive electrode of the electrode contains a compound represented by $NaFeO_2$, $NaNiO_2$, $NaCoO_2$, $NaMnO_2$, $NaFe_{1-x}M^1{}_xO_2$, $NaNi_{1-x}M^1{}_xO_2$, $NaCo_{1-x}M^1{}_xO_2$, or $NaMn_{1-x}M^1{}_xO_2$ wherein $M^1$ represents one or more elements selected from the group consisting of trivalent metals, and $0 \leq x < 0.5$.

10. The battery according to any one of Claims 2 to 7, wherein the electrode containing the carbonaceous material is a positive electrode.

**11.** The battery according to any one of Claims 2 to 10, comprising a laminated porous film present between a positive electrode and a negative electrode, the laminated porous film having been obtained by laminating a heat resistant porous layer containing a heat resistant resin and a shutdown layer containing a thermoplastic resin.

**12.** Use of a carbonaceous material obtained by a production method comprising a step of heating a compound of said formula (1) or a calcination product of the compound, under an inert gas atmosphere in the range of from 200°C to 3000°C, for an electrode of a sodium ion secondary battery.

**13.** Use of a compound of said formula (1) or a calcination product of the compound, for producing an electrode composed of a carbonaceous material for a sodium ion secondary battery.

**14.** A carbonaceous material obtained by a production method comprising a step of heating a compound of said formula (1) or a calcination product of the compound, under an inert gas atmosphere in the range of from 1100°C to 2700°C.

**15.** The material according to Claim 14, wherein R' in said formula (1) represents hydrogen.

**16.** The material according to Claim 14 or 15, wherein the carbonaceous material has a total content of alkali metals and alkaline earth metals of 100 ppm or less (by weight).

**17.** The material according to any one of Claims 14 to 16, wherein the compound of said formula (1) or the calcination product of the compound is a calcination product obtained by heating the compound in the presence of an oxidizing gas at 400°C or lower.

**18.** An electrode containing the material according to any one of Claims 14 to 17.

**19.** Use of a carbonaceous material obtained by a production method comprising a step of heating a compound of said formula (1) or a calcination product of the compound, under an inert gas atmosphere in the range of from 1100°C to 2700°C, for an electrode.

**20.** A method of producing a carbonaceous material, comprising a step of heating a compound of said formula (1) or a calcination product of the compound, under an inert gas atmosphere in the range of from 1100°C to 2700°C.

FIG. 1

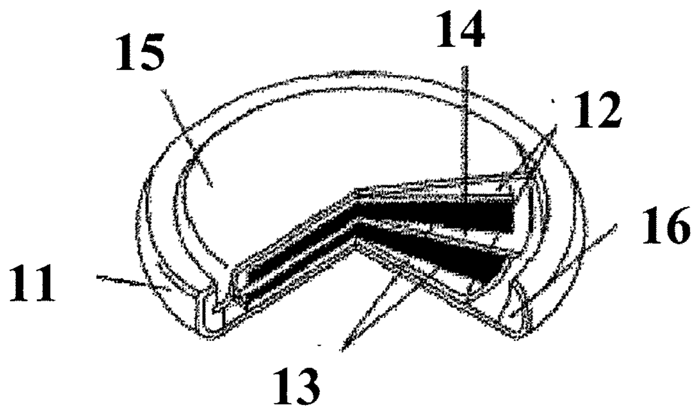

FIG. 2

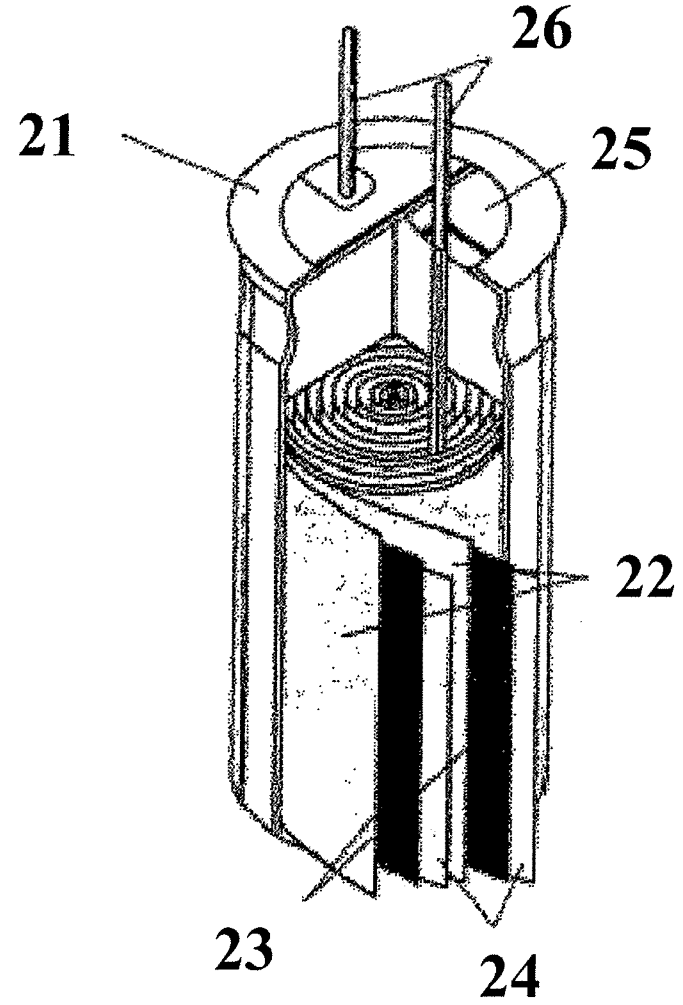

FIG. 3

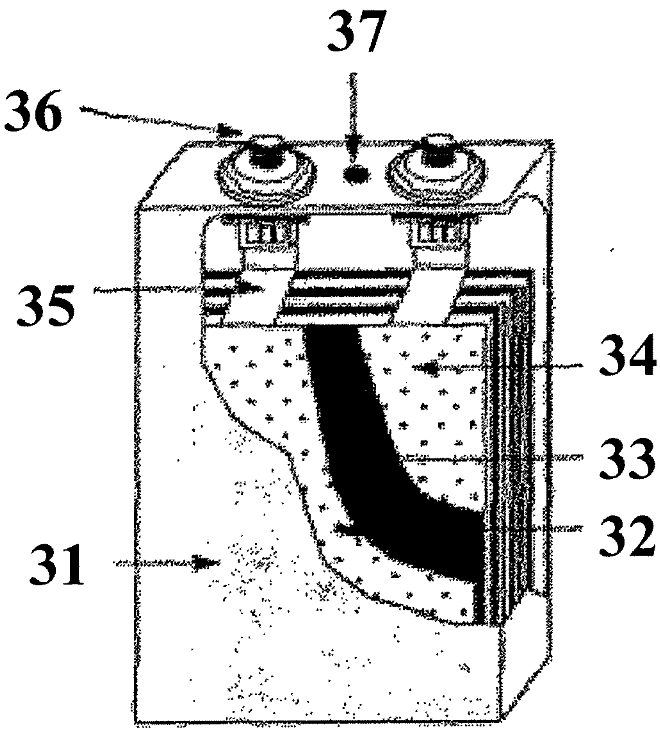

FIG. 4

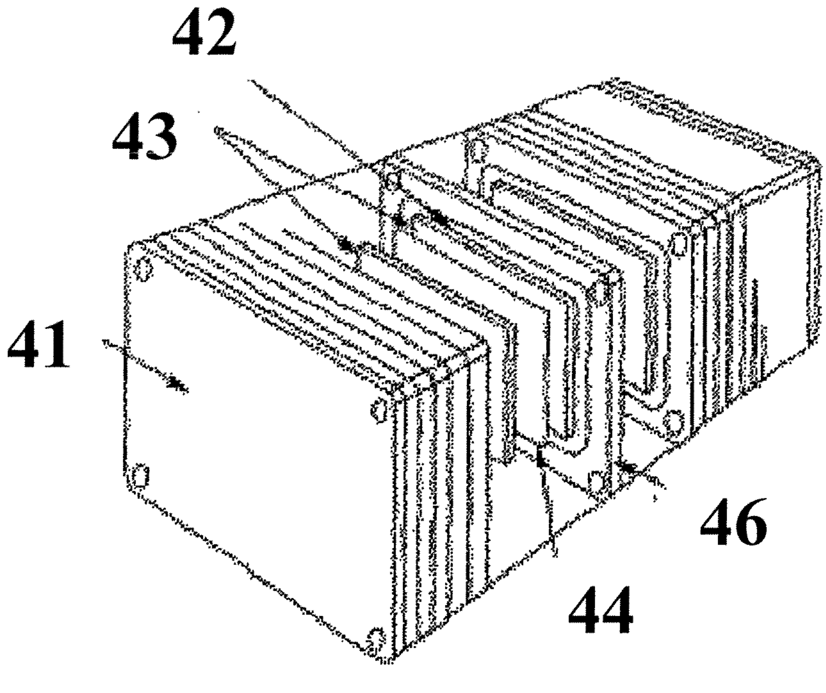

FIG. 5

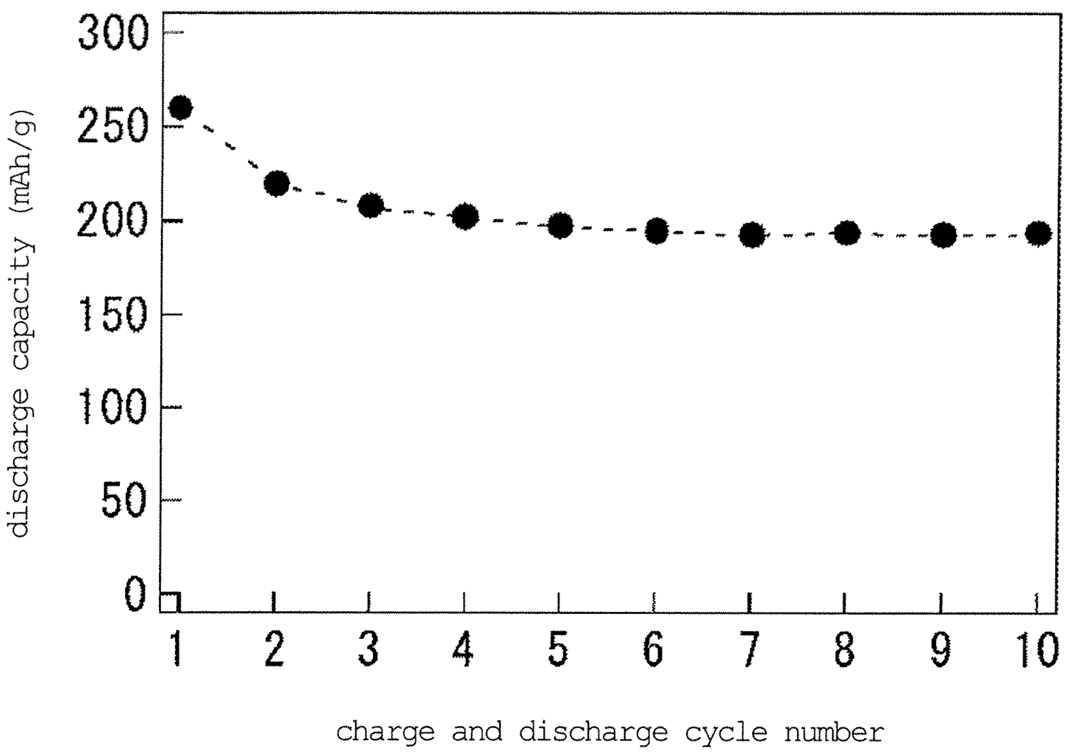
discharge capacity (mAh/g)
300
250
200
150
100
50
0
1
2
3
4
5
6
7
8
9
10
charge and discharge cycle number

FIG. 6

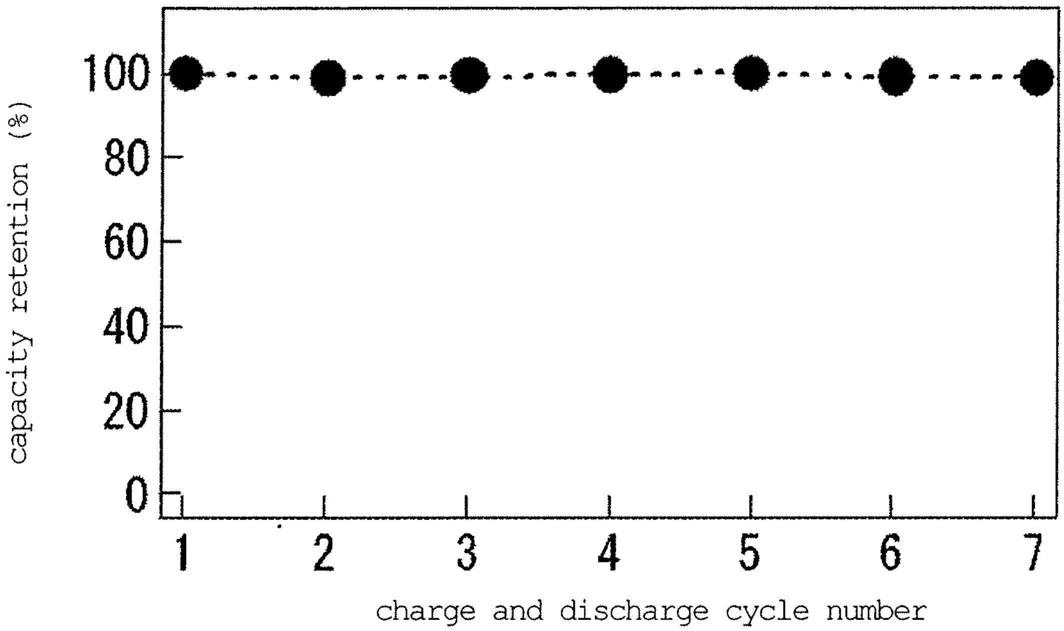
100
80
60
40
20
0
capacity retention (%)
1
2
3
4
5
6
7
charge and discharge cycle number

**INTERNATIONAL SEARCH REPORT**

International application No.
PCT/JP2008/069827

A. CLASSIFICATION OF SUBJECT MATTER
*H01M4/58*(2006.01)i, *H01M4/02*(2006.01)i, *H01M4/50*(2006.01)i, *H01M4/52*(2006.01)i, *H01M10/40*(2006.01)i

According to International Patent Classification (IPC) or to both national classification and IPC

B. FIELDS SEARCHED

Minimum documentation searched (classification system followed by classification symbols)
H01M4/58, H01M4/02, H01M4/50, H01M4/52, H01M10/40

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P,A<br>P,X | JP 2008-100883 A (Sumitomo Chemical Co., Ltd.), 01 May, 2008 (01.05.08), Full text (Family: none) | 1-13<br>14-20 |
| A | JP 11-130414 A (Sumitomo Durez Co., Ltd.), 18 May, 1999 (18.05.99), Claims; Par. Nos. [0010] to [0016] (Family: none) | 1-20 |
| A | JP 3-153512 A (Sumitomo Chemical Co., Ltd.), 01 July, 1991 (01.07.91), Claims; page 2, upper right column, line 2 to page 4, upper right column, line 2 (Family: none) | 1-20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 December, 2008 (19.12.08) | 06 January, 2009 (06.01.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/069827

C (Continuation). DOCUMENTS CONSIDERED TO BE RELEVANT

| Category* | Citation of document, with indication, where appropriate, of the relevant passages | Relevant to claim No. |
|---|---|---|
| A | JP 61-277165 A (Sumitomo Chemical Co., Ltd.), 08 December, 1986 (08.12.86), Claims; page 2, upper left column, line 17 to page 3, lower left column, line 15 (Family: none) | 1-20 |
| A | JP 4-79155 A (Matsushita Electric Industrial Co., Ltd.), 12 March, 1992 (12.03.92), Claims; page 2, lower left column, line 1 to page 3, upper left column, line 3 (Family: none) | 1-20 |
| A | JP 2007-42571 A (Hitachi Chemical Co., Ltd.), 15 February, 2007 (15.02.07), Claims; Par. Nos. [0019] to [0055] (Family: none) | 1-20 |
| A | WO 93/10566 A1 (Honda Motor Co., Ltd.), 27 May, 1993 (27.05.93), Claims; page 4, upper left column, line 24 to page 6, lower left column, line 20 & JP 2561620 B & US 5591545 A1 & EP 0567658 A1 | 1-20 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3155062 A
- JP 2002359155 A
- JP 2002267860 A
- JP 2000030686 A
- JP 10324758 A
- JP 2004172346 A
- JP 2004303567 A
- JP 2003068580 A
- JP 2003257240 A


### Non-patent literature cited in the description


- **P. Timmerman et al.** *Tetrahedron,* 1996, vol. 52, 2663-2704